# EUROPEAN PATENT SPECIFICATION

(11) **EP 1 922 081 B1**
(45) Date of publication and mention of the grant of the patent: **15.08.2012**
(21) Application number: 06779635.9
(22) Date of filing: 08.09.2006
(51) Int. Cl.: A61K 38/21, A61M 15/00, A61P 11/06

(54) **INTERFERON LAMBDA THERAPY FOR TREATMENT OF RESPIRATORY DISEASES**
INTERFERON-LAMBDA-THERAPIE ZUR BEHANDLUNG VON ATEMWEGSERKRANKUNGEN
THERAPIE PAR INTERFERON LAMBDA POUR LE TRAITEMENT DE MALADIES RESPIRATOIRES

(30) Priority: 09.09.2005 GB 0518425; 17.03.2006 US 783297 P
(43) Date of publication of application: 21.05.2008
(73) Proprietor: Imperial Innovations Limited, Exhibition Road London SW7 2PG (GB)
(72) Inventor: DAVIES, Donna, Elizabeth, Wiltshire SP4 6EN (GB); WARK, Peter, Alexander, Blanch, Newcastle, NSW 2322 (AU); HOLGATE, Stephen, Hampshire SO51 7JJ (GB); JOHNSTON, Sebastian, L., London SW3 6PT (GB)
(74) Representative: Williams, Richard Andrew Norman
(86) International application number: PCT/GB2006/050281
(87) International publication number: WO 2007/029041

(56) References cited:
- WO-A2-2005/087253
- US-A1- 2004 037 809
- CONTOLI MARCO ET AL: "Role of deficient type III interferon-lambda production in asthma exacerbations." NATURE MEDICINE. SEP 2006, vol. 12, no. 9, September 2006 (2006-09), pages 1023-1026, XP002414169 ISSN: 1078-8956
- BARTLETT NATHAN W ET AL: "Murine interferon lambdas (type III interferons) exhibit potent antiviral activity in vivo in a poxvirus infection model" JOURNAL OF GENERAL VIROLOGY, vol. 86, no. Part 6, June 2005 (2005-06), pages 1589-1596, XP009076895 ISSN: 0022-1317
- SHEPPARD PAUL ET AL: "IL-28, IL-29 and their class II cytokine receptor IL-28R" NATURE IMMUNOLOGY, NATURE PUBLISHING GROUP,, GB, vol. 4, no. 1, January 2003 (2003-01), pages 63-68, XP002261756 ISSN: 1529-2908
- KOTENKO S V ET AL: "IFN-LAMBDAS MEDIATE ANTIVIRAL PROTECTION THROUGH A DISTINCT CLASS II CYTOKINE RECEPTOR COMPLEX" NATURE IMMUNOLOGY, NATURE PUBLISHING GROUP,, GB, vol. 4, no. 1, January 2003 (2003-01), pages 69-77, XP008040607 ISSN: 1529-2908
- WARK P A B ET AL: "ASTHMATIC BRONCHIAL EPITHELIAL CELLS HAVE A DEFICIENT INNATE IMMUNE RESPONSE TO INFECTION WITH RHINOVIRUS" JOURNAL OF EXPERIMENTAL MEDICINE, TOKYO, JP, vol. 201, no. 6, 21 March 2005 (2005-03-21), pages 937-947, XP009063234 ISSN: 0022-1007 cited in the application
- COCCIA ELIANA M ET AL: "Viral infection and Toll-like receptor agonists induce a differential expression of type I and lambda interferons in human plasmacytoid and monocyte-derived dendritic cells." EUROPEAN JOURNAL OF IMMUNOLOGY, vol. 34, no. 3, March 2004 (2004-03), pages 796-805, XP009076892 ISSN: 0014-2980
- OSTERLUND P ET AL: "GENE EXPRESSION AND ANTIVIRAL ACTIVITY OF ALPHA/BETA INTERFERONS AND INTERLEUKIN-29 IN VIRUS-INFECTED HUMAN MYELOID DENDRITIC CELLS" JOURNAL OF VIROLOGY, THE AMERICAN SOCIETY FOR MICROBIOLOGY, US, vol. 79, no. 15, August 2005 (2005-08), pages 9608-9617, XP009062684 ISSN: 0022-538X
- MEAGER A ET AL: "Biological activity of interleukins-28 and -29: Comparison with type I interferons" CYTOKINE, ACADEMIC PRESS LTD, PHILADELPHIA, PA, US, vol. 31, no. 2, 21 July 2005 (2005-07-21), pages 109-118, XP004962345 ISSN: 1043-4666

## Description

### Field of the invention

The present invention relates to medical uses of interferon lambda (IFN-λ). More particularly, for example, it relates to use of IFN-λ to treat viral-induced exacerbation of respiratory disorders, especially for example, viral-induced exacerbation of asthma by viruses such as rhinovirus (RV), respiratory syncytial virus (RSV) and influenza virus.

### Background of the invention

The majority of acute exacerbations of both asthma and chronic obstructive pulmonary disease (COPD) are precipitated by respiratory virus infection. Respiratory virus infection is a major trigger of exacerbations of asthma in both adults and children. It is implicated in around 80% of paediatric asthma attacks (Johnston et al. BMJ (1995) 310, 1225-1229) and 75% of adult asthma attacks (Wark et al. Eur. Resp. J 2002 19,68-75) It is a significant cause of asthma morbidity and mortality (Campbell et al., BMJ (1997), 1012). The present invention stems from investigation of interferon production in bronchial epithelial cells and bronchoalveolar lavage cells of asthmatics in response to viral infection.

Type I interferons are a family of closely related glycoproteins comprised of thirteen IFN-α subtypes as well as IFN-β, IFN-κ, IFN-τ and IFN-ω The different human IFN-α subtypes have been identified by analysis of human cDNA libraries and by protein analysis of the IFNs produced by stimulated lymphoblastoid cells; the reasons for their heterogeneity remain unclear. Early studies indicated that all subtypes bind the same receptor from which it was inferred that they must elicit identical responses. Subsequently, comparative studies of both purified and recombinant subtypes revealed a spectrum of anti-viral, anti-proliferative and immunomodulatory responses.

There is one type II interferon IFN-gamma, which binds a different receptor and has largely distinct function from the type I IFNs.

Type-I interferons are a very important component of the innate immune response to respiratory virus infection. The method of protection involves initial release of interferon-β which then stimulates further release of interferon-β and of the interferon-αs in a cascade mediated via the type-1 interferon receptor.

The interferon-λs are three closely related proteins which have more recently been discovered (Kotenko S.V. et al, Nature Immunology 2003;Vol 4,69-77; Sheppard P et al, Nature Immunology 2003; Vol 4,63-88). Interferon λ-1 is also known as IL-29, while Interferon λ-2 and 3 are known as IL-28a/b. These interferons bind a third receptor distinct from those of type I or type II interferons. Thus they are now termed the type III interferons. These interferons have been shown to have anti-viral activity in *in vitro* cell studies (see, for example, WO 2004/037995 of Zymogenetics Inc). However, their utility in protecting against any natural respiratory virus infection in humans has not previously been established.

It is worthy of note in this connection that IFN-β-ser has proved ineffective in trial for prophylaxis of natural colds despite its previously reported anti-viral activity (Sperber et al., J. Infect. Dis (1989) 160, 700-705). This may be due to the innate capacity of normal cells to produce IFN-β in response to rhinovirus infection. Equally, it is not possible to extrapolate from *in vitro* studies with IFN-λ showing anti-viral activity that the same interferon type will have any therapeutic value against *in vivo* natural respiratory virus infection.

Interestingly, investigation of interferon production by human asthmatic bronchial epithelial cells in response to rhinovirus infection firstly showed that such cells have a deficient type I interferon response in keeping with observed resistance to early apoptosis and increased virion production compared to RV-infected bronchial epithelial cells from healthy controls. Furthermore, provision of IFN-β to RV-infected asthmatic bronchial epithelial cells in culture was shown to cause a significant reduction in infectious virion production. These results laid the foundation for proposed new therapeutic utility of IFN-β in treating rhinovirus-induced exacerbation of asthma (Wark et al. J. Exp. Med. (21 st March 2005) 201, 937-947).

Further results have suggested extrapolation of use of IFN-β equally for treatment of RV-induced exacerbation of COPD, which encompasses a range of conditions, including chronic bronchitis and emphysema. COPD is a progressive disease of the airways that is characterised by a gradual loss of lung function. The symptoms of COPD include chronic cough and sputum production as well as shortness of breath. Cigarette smoking is the most common cause of COPD.

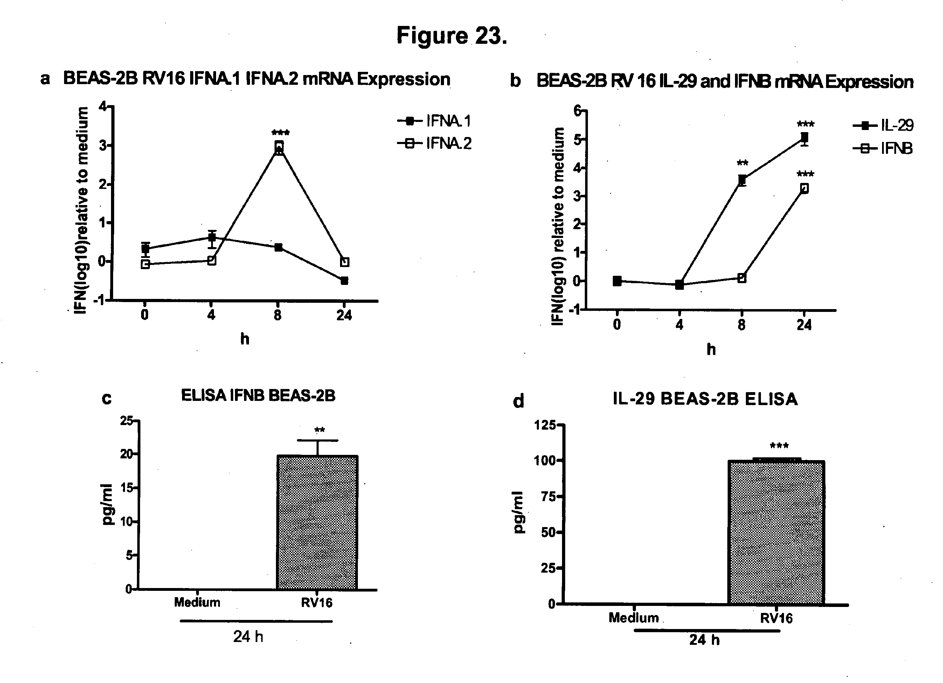

It has now been determined that IFN-λ polypeptides are strongly induced by respiratory virus infections including rhinovirus (the most common) and respiratory syncytial virus (RSV) in human cells. Example 4 and Figures 23 to 26 illustrate such induction in bronchial epithelial cells. Furthermore, the interferon-λs induce β and β also induces λ.

By analysing bronchial epithelial cells and bronchoalveolar lavage cells from asthmatic and normal volunteer patients, it has also now been shown that asthmatic bronchial epithelial cells are additionally deficient in IFN-λ gene expression and protein production when infected with rhinovirus. Such a fmding was not previously shown or contemplated before the present invention and leads to the proposal that administering one or more IFN-λ polypeptides would also constitute an effective therapy for the treatment of viral-induced exacerbation of asthma.

Furthermore, it is suggested that equally IFN-λ polypeptides may be beneficial in the treatment of viral-induced exacerbation of other respiratory disorders such as COPD. By "respiratory disorder", we include in addition to asthma and COPD, allergic bronchopulmonary aspergillosis, eosinophilic pneumonia, allergic bronchitis bronchiectasis, occupational asthma,recative airayd disease syndrome, intersitial lung disease, hyperosinophilic syndrome and parasitic lung disease.

### Summary of the invention

The present invention thus provides 1. An agent selected from: (a) one or more interferon lambda (IFN-λ) polypeptides or (b) a polynucleotide or polynucleotides which express one or more IFN-λ polypeptides in target bronchial epithelial cells, for use in the treatment of viral-induced exacerbation of a respiratory disorder selected from asthma and chronic obstructive pulmonary disease (COPD). . As indicated above, such therapeutic treatment is of especial interest, for example, in alleviating or preventing the problems of viral-induced exacerbation of asthma, most commonly RV-induced exacerbation of asthma but also such exacerbation by, for example, RSV or influenza virus. The administration of the one or more IFN-λ polypeptides may be directly as a polypeptide or via expression from one or more polynucleotides.

A preferred mode of administration to treat viral-induced exacerbation of a respiratory disorder, is by airway delivery medicament, e.g. by use of an aerosol nebuliser. The individual treated may be any animal, but preferably the individual treated will be a human, for example, preferably an asthmatic human.

Described herein is also a device containing a pharmaceutical composition comprising a therapeutic agent which is (i) one or more IFN-λ polypeptides or (ii) one or more polynucleotides capable of expressing one or more IFN-λ polypeptides as noted above, said device being suitable for airway delivery of said composition. Such a composition may be supplemented with an additional therapeutic agent used to treat the respiratory disorder for simultaneous, separate or sequential administration Thus, the additional therapeutic agent may be formulated to provide a single composition or provided in a separate composition. Products suitable for such administration regimes constitute a still further aspect of the invention.

As a preferred embodiment, there is provided 7. An agent for use according to any one of claims 1 to 6, As a combined product with an inhaled corticosteroid, wherein said agent and said corticosteroid are to be administered simultaneously, separately or sequentially to treat viral exacerbation of a respiratory disorder selected from asthma and COPD.

As a consequence of the studies reported herein, it is additionally postulated that IFN-λ polypeptides may be of benefit in relation to an allergic disorder such as asthma, independent of any viral exacerbation. It is well established that the prevalence of asthma is increasing as is the prevalence of allergic diseases in general. This increase in prevalence has been suggested to be related to an absence of infectious disease, in that those with a high exposure to infectious disease early in life have a very low risk of developing asthma and allergies later in life. It is extrapolated that both IFN-λs and IFN-β may be used as a preventative treatment by mimicking the protective role of infection.

Allergic disorders, including asthma, rhinitis, eczema, food allergies and anaphylaxis are thought to be related to impaired TH1 immune responses which themselves are a consequence of impaired type I interferon responses, both the consequence of inadequate exposure to infectious disease early in life. Data presented herein suggests that administering IFN-λs early in life could mimic the protective effect of infectious disease, promote type I interferon and TH1 immune responses and prevent the development of TH2 driven allergic disorders. This preventive therapy could be administered early in life but IFN-λs might also be administered later in life to treat/cure allergic disorder, in other words to reverse the TH2 driven sensitisation and immune responses to allergens.

### Detailed description of the invention

As indicated above, a preferred use of IFN-λ polypeptides now presented is, however, to alleviate or prevent viral-induced exacerbation of asthma, especially in humans. Such viral-induced exacerbation will most commonly be the result of RV-infection. However, the invention is equally applicable to asthma exacerbation by other viral infections including RSV infection and influenza infection.

Methods of diagnosing whether a patient has or is suffering from a respiratory disorder are well known in the art. For example, guidelines for diagnosing asthma are provided by the Global Initiative for Asthma (GINA) as part of a publication titled: "Pocket guide for asthma prevention and management", which is available from www.ginasthma.com. Similarly, guidelines for diagnosing COPD are provided by the Global Initiative for Obstructive Lung Disease (GOLD) as part of a publication titled: "Pocket guide to COPD diagnosis, management and prevention: a guide for heath care professionals", which is available from www.goldcopd.com. Relevant extracts from both of these documents are provided in the accompanying examples.

A use according to the invention may comprise administering one IFNλ polypeptide or the patient may be administered a mixture of IFNλ-1 and IFNλ-2, or a mixture of IFNλ-1 and IFNλ-3, or a mixture of IFNλ-2 and IFNλ-3, or a mixture of IFNλ-1, IFNλ-2 and IFNλ-3.

By "IFNλ polypeptide" we include those polypeptides disclosed in GenBank accession numbers Q8IU54, Q8IZJ0, Q8IZI9, and set out below:

maaawtvvlv tlvlglavag pvptskpttt gkgchigrfk slspqelasf kkardalees
lklknwscss pvfpgnwdlr llqvrerpva leaelaltlk vleaaagpal edvldqplht
lhhilsqlqa ciqpqptagp rprgrlhhwl hrlqeapkke sagcleasvt fnlfrlltrd
lkyvadgnlc lrtsthpest
(IFNλ-1)

mkldmtgdct pvlvlmaavl tvtgavpvar lhgalpdarg chiaqfksls pqelqafkra
kdaleeslll kdcrchsrlf prtwdlrqlq vrerpmalea elaltlkvle atadtdpalv
dvldqplhtl hhilsqfrac iqpqptagpr trgrlhhwly rlqeapkkes pgcleasvtf
nlfrlltrdl ncvasgdlcv
(IFNλ-2)

mkldmtgdcm pvlvlmaavl tvtgavpvar lrgalpdarg chiaqfksls pqelqafkra
kdaleeslll kdckcrsrlf prtwdlrqlq vrerpvalea elaltlkvle atadtdpalg
dvldqplhtl hhilsqlrac iqpqptagpr trgrlhhwlh rlqeapkkes pgcleasvtf
nlfrlltrdl ncvasgdlcv
(IFNλ-3)

By "IFNλ polypeptide" is included any full length naturally occurring IFNλ polypeptide or fragment thereof, or any variant thereof.

"Fragments" or "variants" of an IFNλ polypeptide are those which have substantially the same or more biological activity of IFNλ polypeptide so as to be useful as therapeutic agents in the method of the invention. Such variants and fragments will usually include at least one region of at least five consecutive amino acids which has at least 90% homology with the most homologous five or more consecutive amino acids region of the said polypeptide. A fragment is less than 100% of the whole polypeptide.

The biological activity of "fragments" or "variants" of an IFNλ polypeptide may be determined by, for example, measuring the anti-viral activity of such a polypeptide against RV infection in bronchial epithelial cells, as described in Example 1 below. By "substantially the same or more" we include where the "fragments" or "variants" of an IFNλ polypeptide has at least 50%, 60%, 70%, 80%, 90%, 95%, 100% or more of the biological activity of IFNλ polypeptide.

It will be recognised by those skilled in the art that the IFNλ polypeptides may be modified by known polypeptide modification techniques. These include the techniques disclosed in US Patent No 4,302,386 issued 24 November 1981 to Stevens. Such modifications may enhance biological activity to be useful as therapeutic agents. For example, a few amino acid residues may be changed. Unwanted sequences can be removed by techniques well known in the art. For example, the sequences can be removed via limited proteolytic digestion using enzymes such as trypsin or papain or related proteolytic enzymes.

Thus, the IFNλ polypeptides of use in the invention include modified polypeptides, including synthetically derived polypeptides or fragments of the original polypeptide.

The IFNλ polypeptide may be prepared from a number of different sources. For example, recombinant IFNλ polypeptide can be expressed in a cell using a number of different expression systems (both prokaryotic or eukaryotic) and isolated, optionally with a protein tag. Recombinant IFNλ polypeptide may be secreted into a supernatant and the recombinant polypeptide may then be purified from the supernatant.

Methods by which recombinant polypeptide can be expressed and purified from cells are well known in the art and are routine procedure which can be performed by the skilled person. Such methods are disclosed in, for example, and are provided in, for example, Sambrook et al., Molecular Cloning: A Laboratory Manual. 2001. 3rd edition.

In general, DNA encoding the desired IFNλ polypeptide is expressed in a suitable microbial host cell. Thus, DNA encoding IFNλ polypeptide may be used in accordance with known techniques, appropriately modified in view of the teachings contained herein, to construct an expression vector, which is then used to transform an appropriate host cell for the expression and production of IFNλ polypeptide. Such techniques include those disclosed in US Patent Nos. 4,440,859 issued 3 April 1984 to Rutter et al*,* 4,530,901 issued 23 July 1985 to Weissman, 4,582,800 issued 15 April 1986 to Crowl, 4,677,063 issued 30 June 1987 to Mark et al*,* 4,678,751 issued 7 July 1987 to Goeddel, 4,704,362 issued 3 November 1987 to Itakura et al*,* 4,710,463 issued 1 December 1987 to Murray, 4,757,006 issued 12 July 1988 to Toole, Jr. et al*,* 4,766,075 issued 23 August 1988 to Goeddel et al and 4,810,648 issued 7 March 1989 to Stalker,

The DNA encoding IFNλ polypeptide may be joined to a wide variety of other DNA sequences for introduction into an appropriate host. The companion DNA will depend upon the nature of the host, the manner of the introduction of the DNA into the host, and whether episomal maintenance or integration is desired.

Generally, the DNA is inserted into an expression vector, such as a plasmid, in proper orientation and correct reading frame for expression. If necessary, the DNA may be linked to the appropriate transcriptional and translational regulatory control nucleotide sequences recognised by the desired host, although such controls are generally available in the expression vector. Thus, the DNA insert may be operatively linked to an appropriate promoter. Bacterial promoters include the E.coli *lacI* and *lacZ* promoters, the T3 and T7 promoters, the *gpt* promoter, the phage λ PR and PL promoters, the *phoA* promoter and the *trp* promoter. Eukaryotic promoters include the CMV immediate early promoter, the HSV thymidine kinase promoter, the early and late SV40 promoters and the promoters of retroviral LTRs. Other suitable promoters will be known to the skilled artisan. The expression constructs will desirably also contain sites for transcription initiation and termination, and in the transcribed region, a ribosome binding site for translation. (Hastings *et al,* International Patent No. WO 98/16643, published 23 April 1998).

The vector is then introduced into the host through standard techniques. Generally, not all of the hosts will be transformed by the vector and it will therefore be necessary to select for transformed host cells. One selection technique involves incorporating into an expression vector containing any necessary control elements a DNA sequence marker that codes for a selectable trait in the transformed cell. These markers include dihydrofolate reductase, G418 or neomycin resistance for eukaryotic cell culture, and tetracyclin, kanamycin or ampicillin resistance genes for culturing in *E.coli* and other bacteria. The selectable markers could also be those which complement auxotrophisms in the host. Alternatively, the gene for such a selectable trait can be on another vector, which is used to co-transform the desired host cell.

Host cells that have been transformed by DNA encoding IFNλ polypeptide are then cultured for a sufficient time and under appropriate conditions known to those skilled in the art in view of the teachings disclosed herein to permit the expression of interferon polypeptide.

Many microbial expression systems are known, including systems employing: bacteria (eg. *E.coli* and *B. subtilis*) transformed with, for example, recombinant bacteriophage, plasmid or cosmid DNA expression vectors; yeasts (eg. *Saccaromyces cerevisiae*) transformed with, for example, yeast expression vectors; insect cell systems transformed with, for example, viral expression vectors (eg. baculovirus).

The vectors can include a prokaryotic replicon, such as the Col E1 *ori*, for propagation in a prokaryote. The vectors can also include an appropriate promoter such as a prokaryotic promoter capable of directing the expression (transcription) of the genes in a bacterial host cell, such as *E.coli,* transformed therewith, and a translation initiation sequence, such as the Shine-Dalgarno consensus ribosomebinding sequence, usually adjacent to the promoter sequence, that forms part of the resulting transcript and from which translation of the cloned gene transcript can commence.

A promoter is an expression control element formed by a DNA sequence that permits binding ofRNA polymerase and transcription to occur. Promoter sequences compatible with exemplary bacterial hosts are typically provided in plasmid vectors containing convenient restriction sites for insertion of a DNA segment of the present invention.

Typical prokaryotic vector plasmids are: pUC18, pUC19, pBR322 and pBR329 available from Biorad Laboratories (Richmond, CA, USA); pTrc99A, pKK223-3, pKK233-3, pDR540 and pRIT5 available from Pharmacia (Piscataway, NJ, USA); pBS vectors, Phagescript vectors, Bluescript vectors, pNH8A, pNH16A, pNH18A, pNH46A available from Stratagene Cloning Systems (La Jolla, CA 92037, USA). Preferred prokaryotic vector plasmids include pET26b (Novagen, Nottingham, UK).

Useful yeast plasmid vectors are pRS403-406 and pRS413-416 and are generally available from Stratagene Cloning Systems (La Jolla, CA 92037, USA). Plasmids pRS403, pRS404, pRS405 and pRS406 are Yeast Integrating plasmids (YIps) and incorporate the yeast selectable markers *HIS3, TRPI, LEU2* and *URA3.* Plasmids pRS413-416 are Yeast Centromere plasmids (YCps).

Methods well known to those skilled in the art can be used to construct expression vectors containing the coding sequence and, for example appropriate transcriptional or translational controls. One such method involves ligation via homopolymer tails. Homopolymer polydA (or polydC) tails are added to exposed 3' OH groups on the DNA fragment to be cloned by terminal deoxynucleotidyl transferases. The fragment is then capable of annealing to the polydT (or polydG) tails added to the ends of a linearised plasmid vector. Gaps left following annealing can be filled by DNA polymerase and the free ends joined by DNA ligase.

Another method involves ligation via cohesive ends. Compatible cohesive ends can be generated on the DNA fragment and vector by the action of suitable restriction enzymes. These ends will rapidly anneal through complementary base pairing and remaining nicks can be closed by the action of DNA ligase.

A further method uses synthetic molecules called linkers and adaptors. DNA fragments with blunt ends are generated by bacteriophage T4 DNA polymerase or *E.coli* DNA polymerase I which remove protruding 3' termini and fill in recessed 3' ends. Synthetic linkers, pieces of blunt-ended double-stranded DNA which contain recognition sequences for defined restriction enzymes, can be ligated to blunt-ended DNA fragments by T4 DNA ligase. They are subsequently digested with appropriate restriction enzymes to create cohesive ends and ligated to an expression vector with compatible termini. Adaptors are also chemically synthesised DNA fragments which contain one blunt end used for ligation but which also possess one preformed cohesive end.

Synthetic linkers containing a variety of restriction endonuclease sites are commercially available from a number of sources including International Biotechnologies Inc, New Haven, CN, USA.

A desirable way to modify DNA encoding the IFNλ polypeptide is to use the polymerase chain reaction as disclosed by Saiki et al. (1988) Science 249, 487-491. In this method the DNA to be enzymatically amplified is flanked by two specific oligonucleotide primers which themselves become incorporated into the amplified DNA. The said specific primers may contain restriction endonuclease recognition sites which can be used for cloning into expression vectors using methods known in the art.

Accordingly, the procedures outlined above can be used to prepare a microbial expression system for the preparation of IFNλ polypeptide.

The IFNλ polypeptide can be recovered from microbial expression systems using a number of different well known methods, including ammonium sulphate or ethanol precipitation, acid extraction, anion or cation exchange chromatography, phosphocellulose chromatography, hydrophobic interaction chromatography, affinity chromatography, hydroxylapatite chromatography, lectin chromatography, dyeligand chromatography and reverse phase high performance liquid chromatography ("HPLC").

Such methods may include the step of lysing the microbial host cells (unless the expression system directed the IFNλ polypeptide to be secreted from the cell).

Alternatively, IFN-λ polypeptides may be synthesised by the Fmoc-polyamide mode of solid-phase peptide synthesis as disclosed by Lu et al. (1981) J. Org. Chem. 46, 3433 and references therein. Temporary N-amino group protection is afforded by the 9-fluorenylmethyloxycarbonyl (Fmoc) group. Repetitive cleavage of this highly base-labile protecting group is effected using 20% piperidine in N,N-dimethylformamide. Side-chain functionalities may be protected as their butyl ethers (in the case of serine threonine and tyrosine), butyl esters (in the case of glutamic acid and aspartic acid), butyloxycarbonyl derivative (in the case of lysine and histidine), trityl derivative (in the case of cysteine) and 4-methoxy-2,3,6-trimethylbenzenesulphonyl derivative (in the case of arginine). Where glutamine or asparagine are C-terminal residues, use is made of the 4,4'-dimethoxybenzhydryl group for protection of the side chain amido functionalities. The solid-phase support is based on a polydimethyl-acrylamide polymer constituted from the three monomers dimethylacrylamide (backbone-monomer), bisacryloylethylene diamine (cross linker) and acryloylsarcosine methyl ester (functionalising agent). The peptide-to-resin cleavable linked agent used is the acid-labile 4-hydroxymethyl-phenoxyacetic acid derivative. All amino acid derivatives are added as their preformed symmetrical anhydride derivatives with the exception of asparagine and glutamine, which are added using a reversed N,N-dicyclohexyl-carbodiimide/1-hydroxybenzotriazole mediated coupling procedure. All coupling and deprotection reactions are monitored using ninhydrin, trinitrobenzene sulphonic acid or isotin test procedures. Upon completion of synthesis, peptides are cleaved from the resin support with concomitant removal of side-chain protecting groups by treatment with 95% trifluoroacetic acid containing a 50% scavenger mix. Scavengers commonly used are ethanedithiol, phenol, anisole and water, the exact choice depending on the constituent amino acids of the peptide being synthesised. Trifluoroacetic acid is removed by evaporation *in vacuo,* with subsequent trituration with diethyl ether affording the crude peptide. Any scavengers present are removed by a simple extraction procedure which on lyophilisation of the aqueous phase affords the crude peptide free of scavengers. Reagents for peptide synthesis are generally available from Calbiochem-Novabiochem (UK) Ltd, Nottingham NG7 2QJ, UK. Purification may be effected by any one, or a combination of, techniques such as size exclusion chromatography, ion-exchange chromatography and (principally) reverse-phase high performance liquid chromatography. Analysis of peptides may be carried out using thin layer chromatography, reverse-phase high performance liquid chromatography, amino-acid analysis after acid hydrolysis and by fast atom bombardment (FAB) mass spectrometric analysis.

As indicated above, one or more IFN-λ polypeptides may be administered directly or via expression from one or more polynucleotides. Such a polynucleotide may preferably be in the form of a vector capable of directing expression of the IFN-λ(s) in the bronchial epithelium. Such expression vectors may be any type conventionally considered for gene therapy. They may be plasmid expression vectors administered as naked DNA or complexed with one or more cationic amphiphiles, e.g. one or more cationic lipids, e.g. in the form of DNA/liposomes. A viral vector may alternatively be employed. Vectors for expression of therapeutic proteins in the airways of human lung have previously been described. For example, Published International Application WO 01/91800 (Isis innovation Limied) describes for such purpose expression vectors including the human ubiquitin C promoter. Examples of expression vectros for use in directing transgene expression in airway epithelia have also been described in Chow et al., Proc, Natl. Acad. Sci. USA (1997) 94, 14695-14700.

IFN-λ polypeptides may be formulated together with one or more acceptable carriers to provide a pharmaceutical composition for therapeutic use. The carrier(s) must be "acceptable" in the sense of being compatible with the compound and not deleterious to the recipients thereof. Such carriers are well known in the pharmaceutical art. For the purpose of treatment of a viral-induced exacerbation of a respiratory disorder in accordance with the invention, it is particularly preferred that the IFNλ polypeptide is formulated for airway administration.

For such administration, the IFNλ polypeptide is conveniently delivered in the form of an aerosol spray presentation from a pressurised container, pump, spray or nebuliser with the use of a suitable propellant, e.g. dichlorodifluoromethane, trichlorofluoromethane, dichlorotetrafluoro-ethane, a hydrofluoroalkane such as 1,1,1,2-tetrafluoroethane (HFA 134A3 or 1,1,1,2,3,3,3-heptafluoropropane (HFA 227EA3), carbon dioxide or other suitable gas. In the case of a pressurised aerosol, the dosage unit may be determined by providing a valve to deliver a metered amount. The pressurised container, pump, spray or nebuliser may contain a solution or suspension of the active compound (s), *e.g.* using a mixture of ethanol and the propellant as the solvent, which may additionally contain a lubricant, *e.g.* sorbitan trioleate. The formulation may also be delivered using ultrasonic nebulisation techniques.

Thus, as indicated above, in a further aspect the invention provides a device containing a pharmaceutical composition comprising a therapeutic agent which is (i) one or more IFN-λ polypeptides or (ii) one or more polynucleotides capable of expressing one or more IFN-λ polypeptides as discussed above and suitable for airway delivery of said composition. Such a composition may be supplemented with an additional therapeutic agent used to treat the respiratory disorder for simultaneous, separate or sequential administration. Thus, the additional therapeutic agent may be formulated to provide a single composition or provided in a separate composition. Products suitable for such administration regimes constitute a still further aspect of the invention.

Thus reference has previously been made above to a product for treatment of viral-induced exacerbation of asthma comprising for simultaneous, separate or sequential administration (a) at least one IFN-λ polypeptide or a polynucleotide capable of expressing at least one IFN-λ polypeptide in the bronchial epithelial cells to be targeted and (b) an inhaled corticosteroid, such as, for example, fluticasone, beclomethasone and budesonide. Such a product may be in the form of a single pharmaceutical composition suitable for aerosol delivery to the airways.

It will be appreciated that the overall daily dose with an aerosol will vary from patient to patient, and may be administered in a single dose or in divided doses throughout the day.

An IFNλ polypeptide to be administered for a method of the invention may be derivatised to improve the pharmacokinetic or immunogenic properties of the polypeptide. For example, an IFN-λ polypeptide may be PEGylated and/or conjugated to albumin or a further substance so as to increase stability of the IFN-λ polypeptide.

PEGylation is a method well known to those skilled in the art wherein a polypeptide or peptidomimetic compound is modified such that one or more polyethylene glycol (PEG) molecules are covalently attached to the side chain of one or more amino acids or derivatives thereof. It is one of the most important molecule altering structural chemistry techniques (MASC). Other MASC techniques may be used; such techniques may improve the pharmacodynamic properties of the molecule, for example extending its half life *in vivo.* A PEGprotein conjugate is formed by first activating the PEG moiety so that it will react with, and couple to, the protein or peptidomimetic compound of the invention. PEG moieties vary considerably in molecular weight and conformation, with the early moieties (monofunctional PEGs; mPEGs) being linear with molecualr weights of 12kDa or less, and later moieties being of increased molecular weights. PEG2, a recent innovation in PEG technology, involves the coupling of a 30kDa (or less) mPEG to a lysine animo acid (although PEGylation can be extended to the addition of PEG to other amino acids) that is further reacted to form a branched structure that behaves like a linear mPEG of much greater molecular weight (Kozlowski et al., (2001), Biodrugs 15, 419 - 429). Methods that may be used to covalently attach the PEG molecules to the polypeptide or peptidomimetic compound of the invention are further described in Roberts et al., (2002) Adv. Drug Deliv Rev 54, 459 - 476, Bhadra et al., (2002) Pharmazie 57, 5 - 29, Kozlowski et al., (2001) J Control Release 72, 217 - 224, and Veronese (2001) Biomaterials, 22, 405 - 417 and references referred to therein.

To improve pharmokinetic properties and/or stability, it may additionally or alternatively be chosen to replace naturally-occuring amino acid residues of a natural IFN-λ polypeptide by non-naturally-occurring amino acid residues. Therapeutic proteins such as interferons and growth hormones, in their native state or when recombinantly produced, can be labile molecules exhibiting short shelf-lives, particularly when formulated in aqueous solutions. The instability in these molecules when formulated for administration dictates that the molecules may have to be lyophilized and refrigerated at all times during storage, thereby rendering the molecules difficult to transport and/or store. Storage problems are particularly acute when pharmaceutical formulations must be stored and dispensed outside of the hospital environment. Many protein and peptide drugs also require the addition of high concentrations of other protein such as albumin to reduce or prevent loss of protein due to binding to the container. This is a major concern with respect to proteins, such as interferons.

The role of albumin as a carrier molecule and its inert nature are desirable properties for use as a carrier and transporter of polypeptides *in vivo.* Fusion of albumin to the therapeutic protein may be achieved by genetic manipulation, such that the DNA coding for albumin, or a fragment thereof, is joined to the DNA coding for the therapeutic protein. A suitable host is then transformed or transfected with the fused nucleotide sequences, so arranged on a suitable plasmid as to express a fusion polypeptide. The expression may be effected *in vitro* from, for example, prokaryotic or eukaryotic cells, or *in vivo e.g.* from a transgenic organism.

The invention may be employed for prophylactic or therapeutic purpose. A person could be considered to be at seasonal risk of developing a respiratory viral infection. Thus in the winter there is excess of rhinovirus infections and clear winter epidemics of influenza and RSV. A person with asthma or COPD could be expected to develop symptoms of viral exacerbation upon exposure to a person who has a clear clinical cold. In this case, one or more IFN-λ polypeptides may be administered in accordance with the invention after such exposure to prevent or at least reduce development of viral-exacerbation of the respiratory disorder.

The invention will be understood to be applicable to any viral infection causing viral induced exacerbation of a respiratory disorder associated with deficient IFNλ production in the bronchial epithelium or for preventing such exacerbation. The viral infection may be infection by, for example, any of rhinovirus, RSV or influenza virus. The viral infection may be caused by further respiratory viruses. The invention is particularly preferred for use, however, in treating or preventing rhinovirus-induced exacerbation of a respiratory disorder, especially rhinovirus-induced exacerbation of asthma.

As discussed above, it is now additionally suggested that interferon-λs may be beneficial in the prevention of an allergic disorder such as asthma, independent of their role against virus infections. The data we have generated suggests that administering interferon-λs early in life would mimic the protective effect of infectious disease, promote TH1 immune responses, and prevent the development of TH2 driven allergic sensitisation and allergic disorder. This preventive therapy could be administered early in life, but interferon-λs could also be administered later in life to treat/cure allergic disorder, in other words to reverse the TH2 driven sensitisation and immune responses to allergens.

"Allergic disorder" is a condition associated with a T helper lymphocyte-2 (Th-2) type immune response. In an allergic reaction, high IgE levels occur and Th-2 immune responses predominate over Th-1 responses, resulting in an inflammatory response.

By "allergic disorder" we include allergic sensitisation, allergic rhinitis, eczema, food allergies, anaphylaxis, dermatitis, allergic rhinitis, allergic conjunctivitis, allergic airways disease, hyper-eosinophilic syndrome, contact dermatitis and respiratory diseases characterised by eosinophilic airways inflammation and airway hyperresponsiveness such as allergic asthma, intrinsic asthma, allergic bronchopulmonary aspergillosis, eosinophilic pneumonia, allergic bronchitis bronchiectasis, occupational asthma, reactive airway disease syndrome, interstitial lung disease, hyperosinophilic syndrome or parasitic lung disease. In one aspect, the allergic disease is allergic sensitisation, allergic rhinitis, eczema, food allergies, anaphylaxis, dermatitis, allergic rhinitis, allergic conjunctivitis, hyper-eosinophilic syndrome or contact dermatitis.

A further embodiment is wherein the respiratory disorder is asthma (allergic or intrinsic), chronic obstructive pulmonary disease (COPD), allergic bronchopulmonary aspergillosis, eosinophilic pneumonia, allergic bronchitis bronchiectasis, occupational asthma, reactive airway disease syndrome, interstitial lung disease, hyperosinophilic syndrome or parasitic lung disease. Preferably the respiratory disorder is asthma and/or COPD.

Also described herein is the use of IFNλ polypeptide in the manufacture of a medicament for the prevention or treatment of an allergic disorder.

Preferably, the allergic disorder is an allergic sensitisation, asthma, allergic rhinitis, eczema, food allergies, anaphylaxis, allergic rhinitis, eczema, food allergies, anaphylaxis, dermatitis, allergic rhinitis, allergic conjunctivitis, allergic airways disease, hyper-eosinophilic syndrome, contact dermatitis and respiratory diseases characterised by eosinophilic airways inflammation and airway hyperresponsiveness such as allergic asthma, intrinsic asthma, allergic bronchopulmonary aspergillosis, eosinophilic pneumonia, allergic bronchitis bronchiectasis, occupational asthma, reactive airway disease syndrome, interstitial lung disease, hyperosinophilic syndrome or parasitic lung disease

The invention will now be described by reference to the following, non-limiting Examples and Figures.

### Brief description of the figures

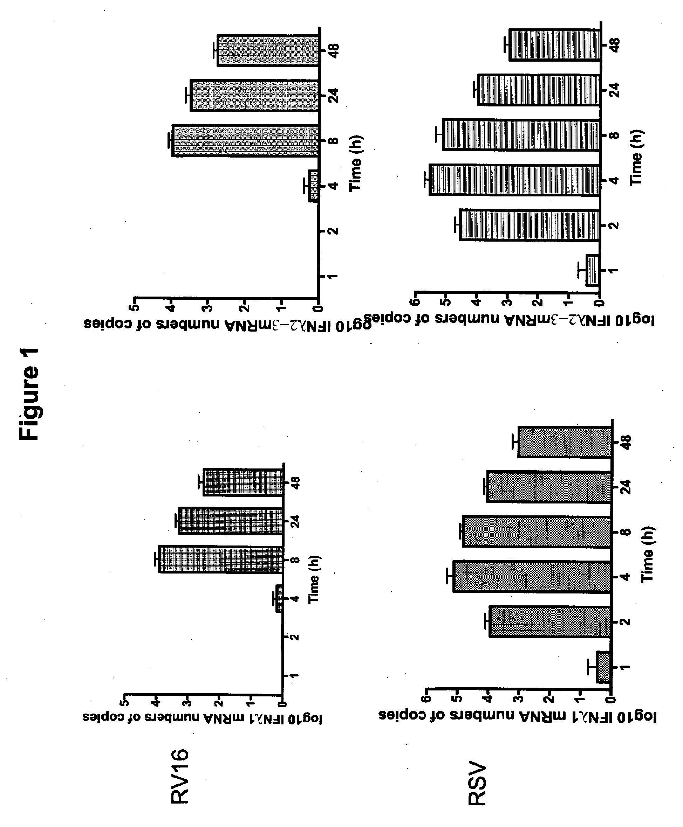

**Figure 1****.** Rhinovirus and RSV both strongly induce interferon lambda mRNA expression in the human bronchial epithelial cell line BEAS2B.

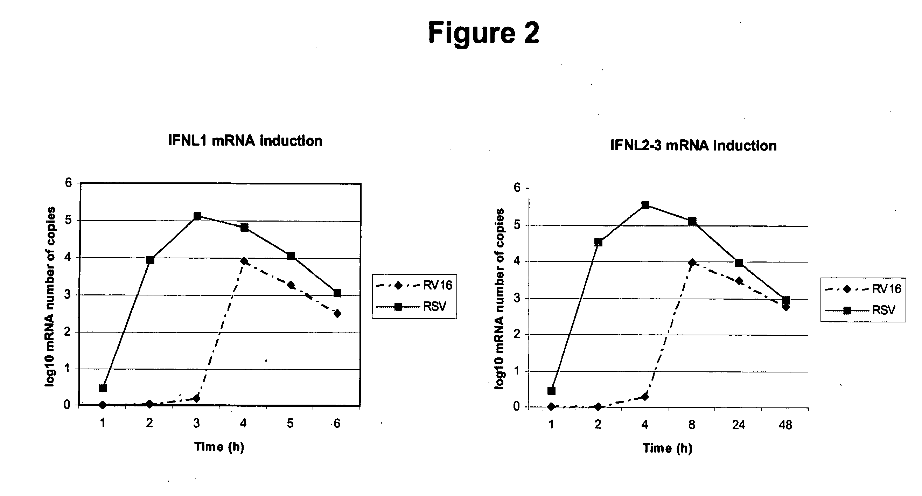

**Figure 2****.** Rhinovirus and RSV both strongly induce interferon lambda mRNA expression in the human bronchial epithelial cell line BEAS2B. Same data as Figure 1.

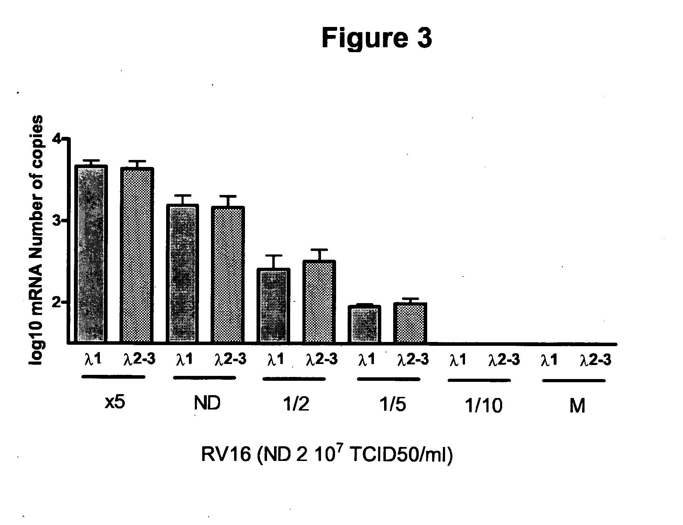

**Figure 3****.** A dose response as stated showing that rhinovirus induction of both IFNλ-1 and IFNλ-2/3 are dose responsive.

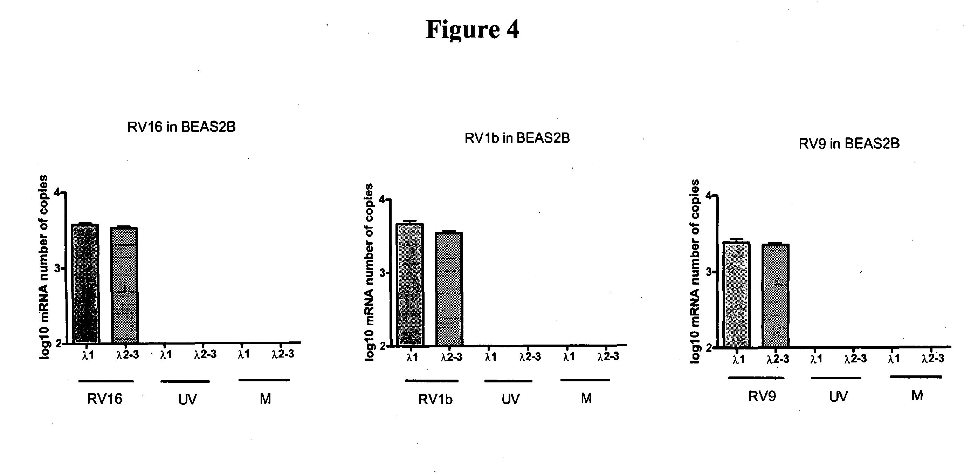

**Figure 4****.** Multiple serotypes of rhinovirus of both major and minor groups induce IFNλs. Since the induction is not observed with UV inactivated viruses, the induction is replication dependent.

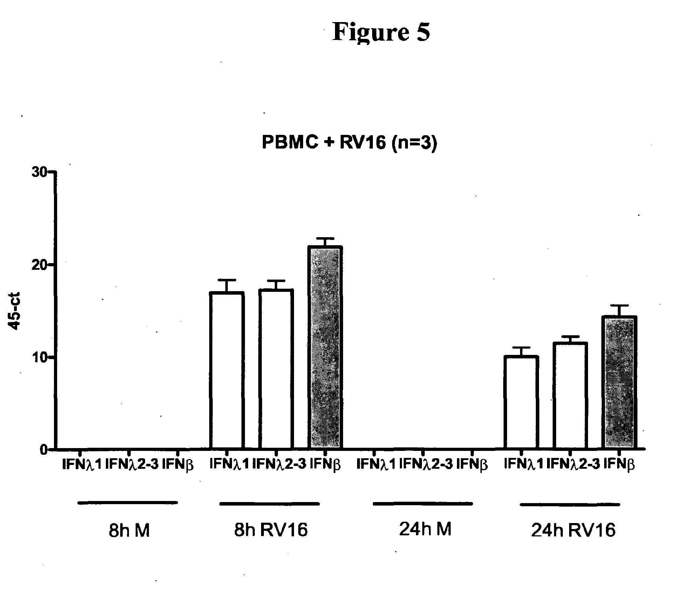

**Figure 5****.** IFNλs are induced from peripheral blood mononuclear cells from healthy volunteers in response to rhinovirus infection, induction peaking at 8 hours but still being significant at 24 hours.

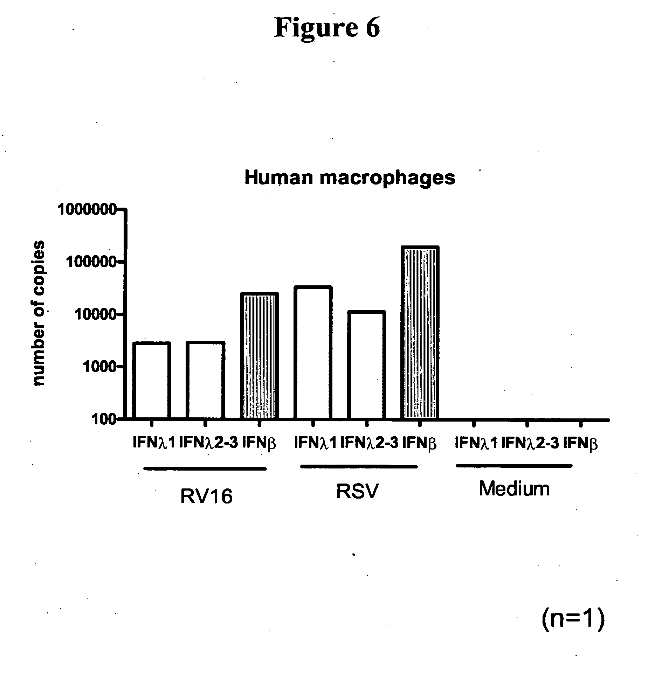

**Figure 6****.** IFNλs are induced from human macrophages by rhinovirus and RSV.

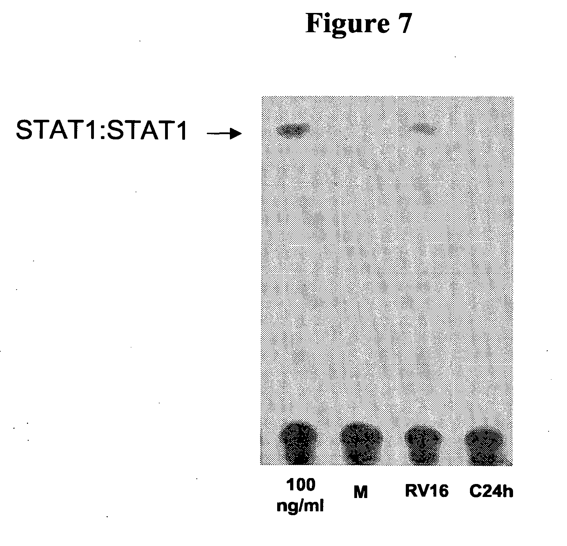

**Figure 7****.** Biological activity with activation of STAT1 by rhinovirus infection. In this experiment supernatants from rhinovirus infected BEAS2B cells were inoculated onto a reporter cell line expressing recombinant lambda receptor and STAT1 activation assessed by gel shift assay. Clear induction of STAT1 activation is observed with supernatants from virus infected bronchial epithelial cells but not control cells.

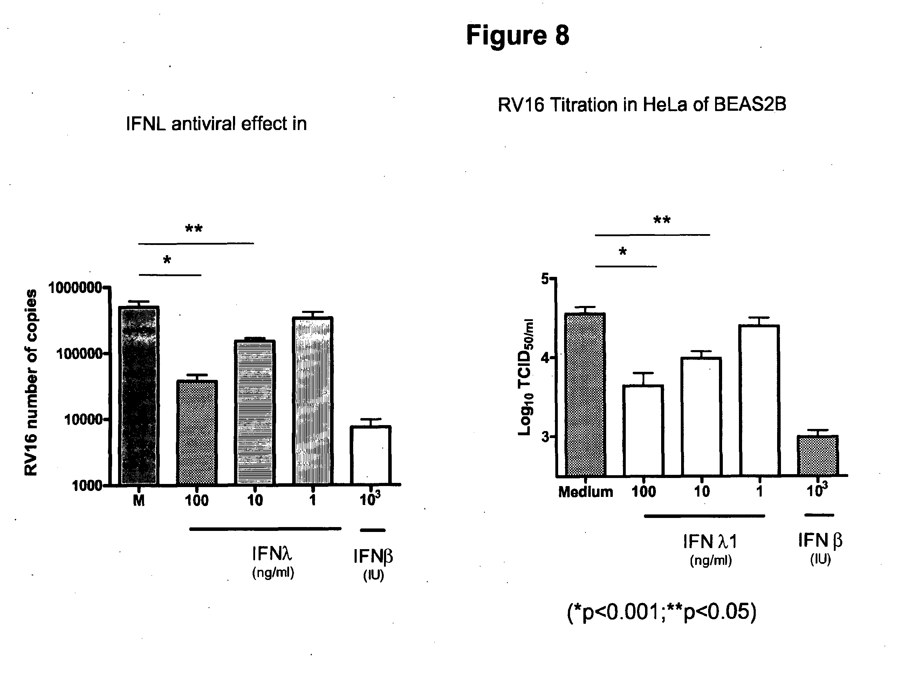

**Figure 8****.** IFNλ-1 has antiviral activity in a dose response manner, reducing rhinovirus 16 viral RNA expression in BEAS2B cells as well as reducing virus release in the supernatant of BEAS2B cells as measured by a HeLa cell titration assay. Viral RNA copy number was assessed by quantitative PCR.

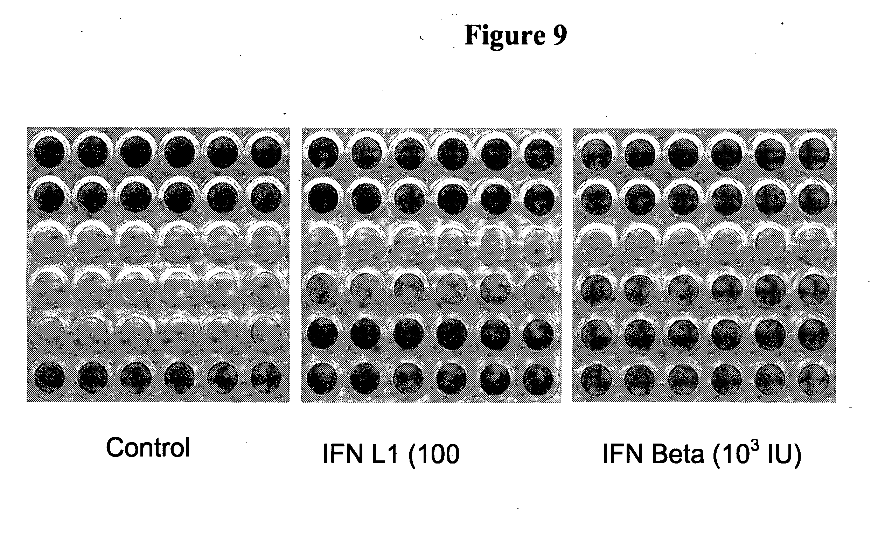

**Figure 9****.** Anti-viral activity in the HeLa cell titration assay showing that virus induced cytopathic effect is inhibited by IFNλ-1 to a similar degree as that observed with interferon β.

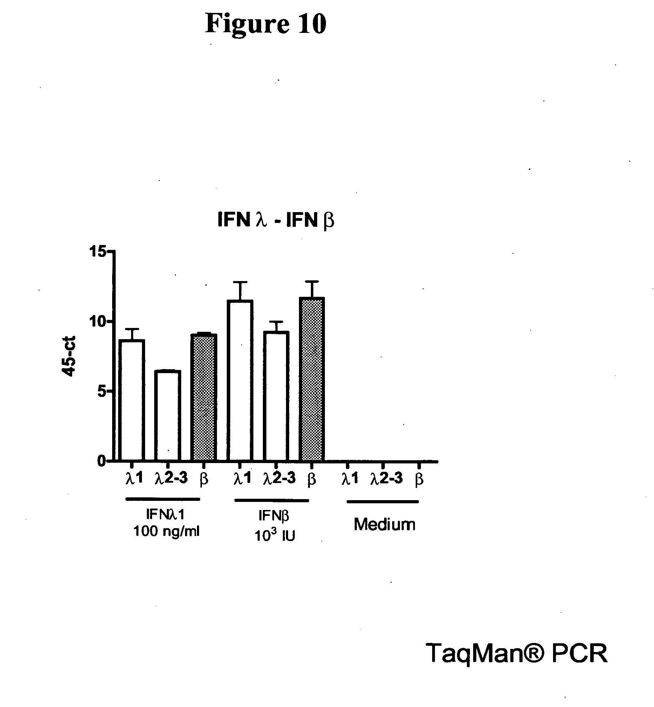

**Figure 10****.** IFNλ-1 induces itself as well as IFNλ 2-3 and interferon β in BEAS2B cells. Similarly interferon β induces itself as well as inducing both IFNλ-1 and IFNλ 2-3. There is thus positive feedback between type 1 and type III interferon sub-types.

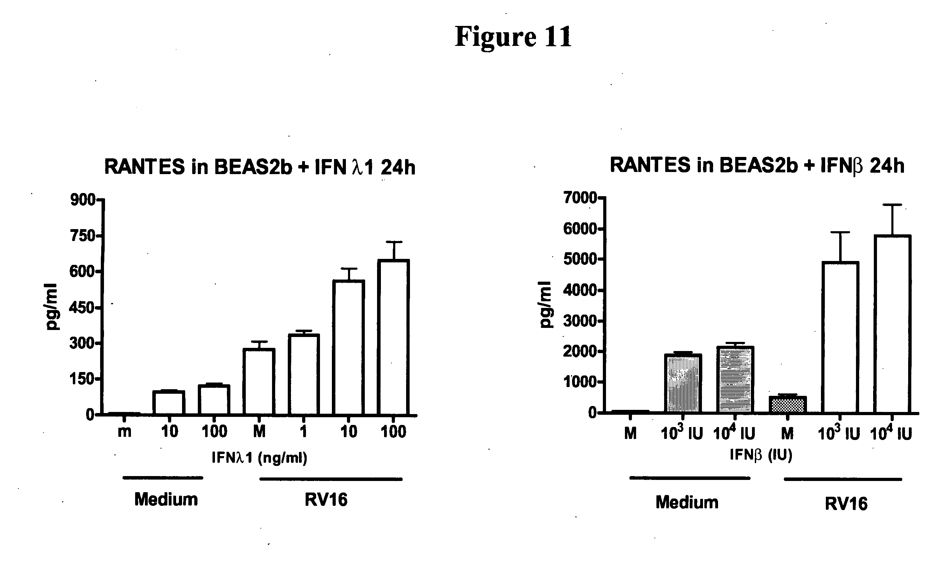

**Figure 11****.** IFNλs induce pro-inflammatory cytokines by themselves in a dose responsive manner and markedly enhance induction of pro-inflammatory cytokines in response to rhinovirus 16 expression, again in a dose responsive manner. These properties are observed in BEAS2B cells and indicate that IFNλs augment responses likely to recruit other inflammatory cells to virus infected epithelium. The right panel shows the same for interferon β.

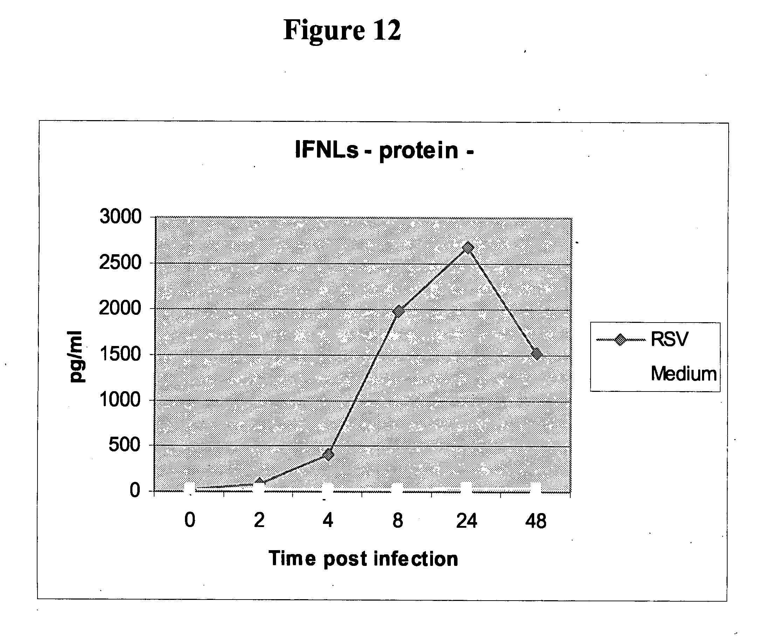

**Figure 12****.** RSV infection of BEAS2B cells results in increased IFNλ protein release into the supernatant in a time responsive manner, peaking at 24 hours.

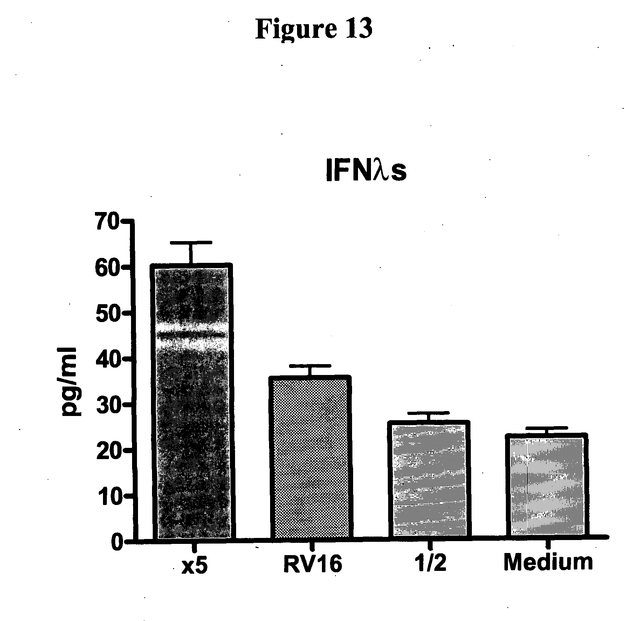

**Figure 13****.** Rhinovirus infection of BEAS2B cells also induces IFNλ protein release into the supernatants of BEAS2B cells in a dose responsive manner.

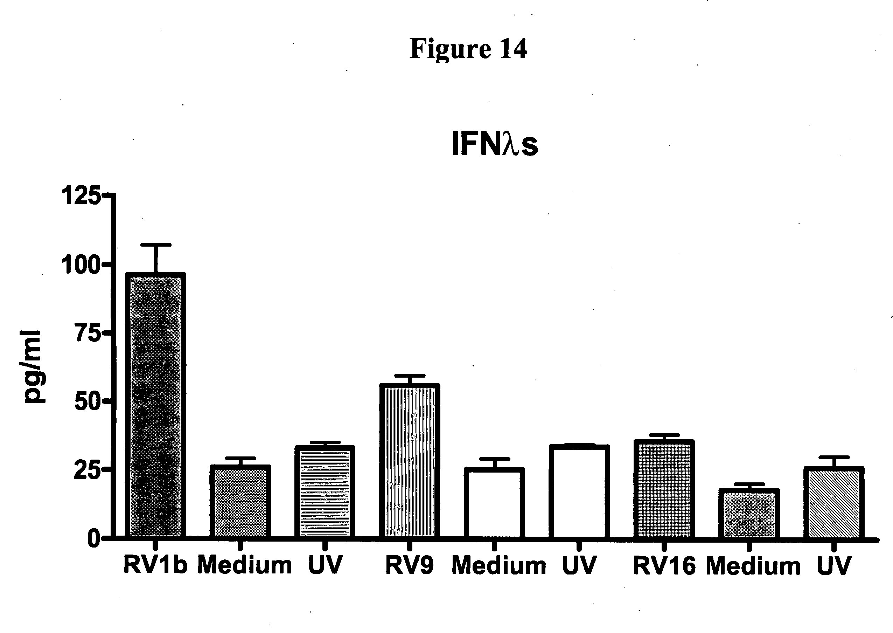

**Figure 14****.** Multiple serotypes of rhinovirus result in release of IFNλ proteins into supernatants of BEAS2B cells. Again, both major and minor serotypes and again in a replication dependent manner.

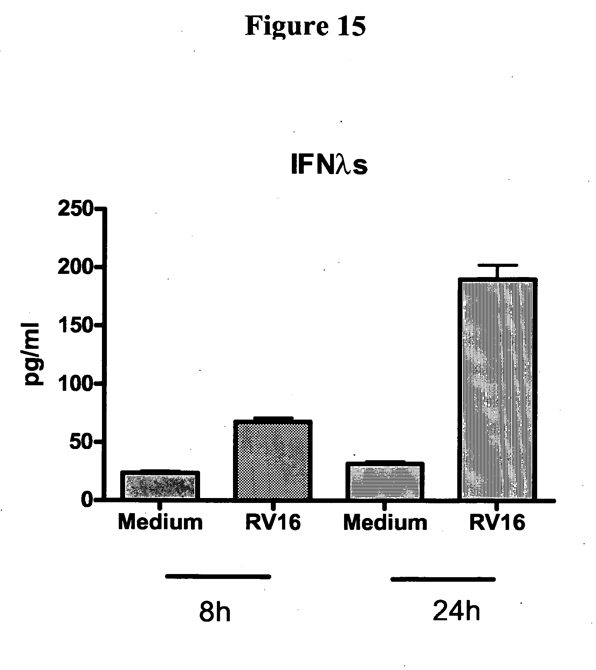

**Figure 15****.** Rhinovirus infection of peripheral blood mononuclear cells from healthy donors leads to an increase in IFNλ protein secretion into supernatants in a time dependent manner.

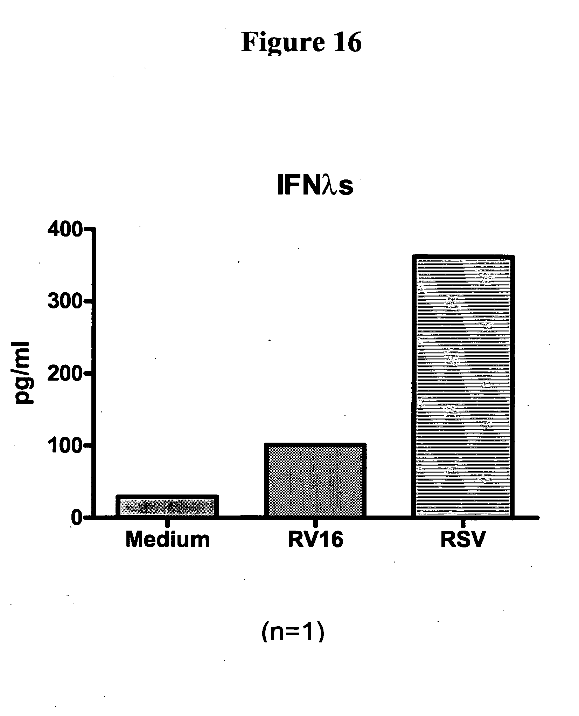

**Figure 16****.** Both rhinovirus and RSV infection of human macrophages results in IFNλ release into supernatants.

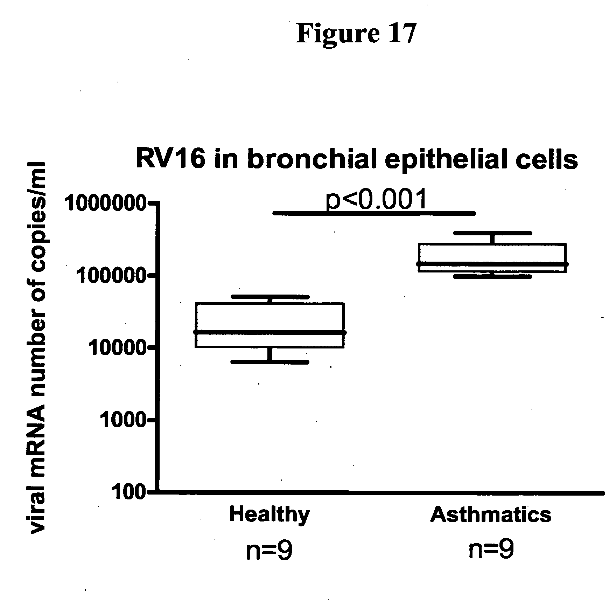

**Figure 17****.** Primary bronchial epithelial cells derived from asthmatic and healthy donors indicate that asthmatic epithelial cells have significantly increased virus replication compared to the normal epithelial cells. The difference in viral RNA copy number was assessed by quantitative PCR. Asthmatic epithelial cells produce more than one log greater viral RNA load than normal epithelial cells.

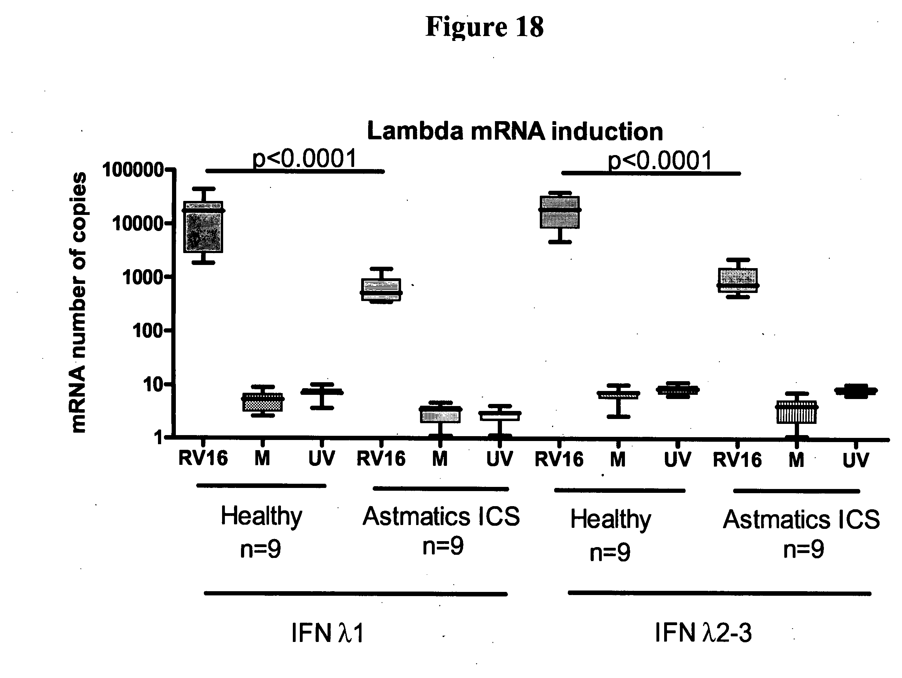

**Figure 18****.** IFNλ mRNA expression in response to rhinovirus infection in healthy and asthmatic epithelial cells is induced. However, induction is deficient in asthmatic relative to normal cells for IFNλs. IFNλ mRNA expression was quantified by quantitative PCR. Induction of IFNλ was replication dependent. Normal volunteers produced more than one log greater amounts of IFNλ mRNA than did asthmatic subjects.

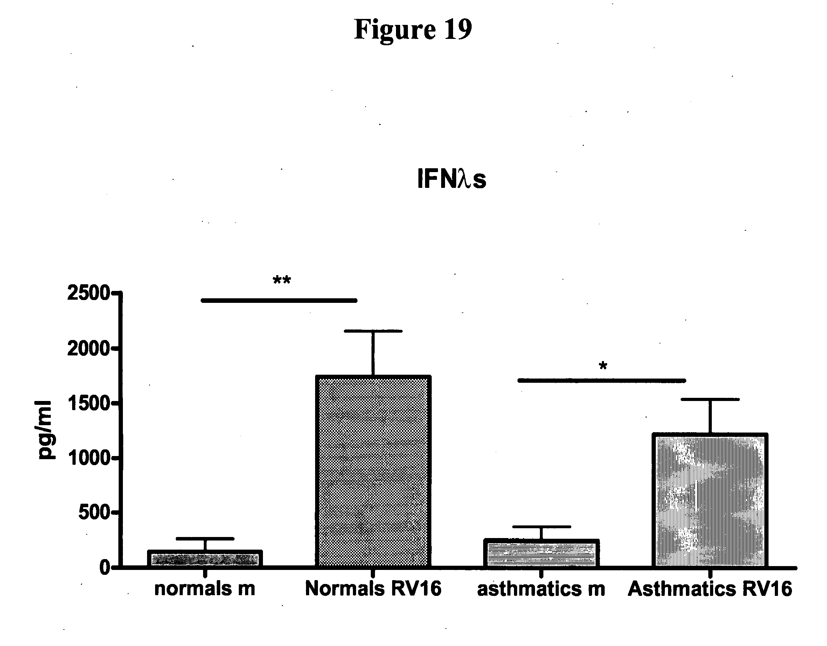

**Figure 19****.** IFNλ protein is induced by rhinovirus infection in both normal and asthmatic bronchial epithelial cells. Levels produced by normal epithelial cells once again are greater than those produced by asthmatic epithelial cells.

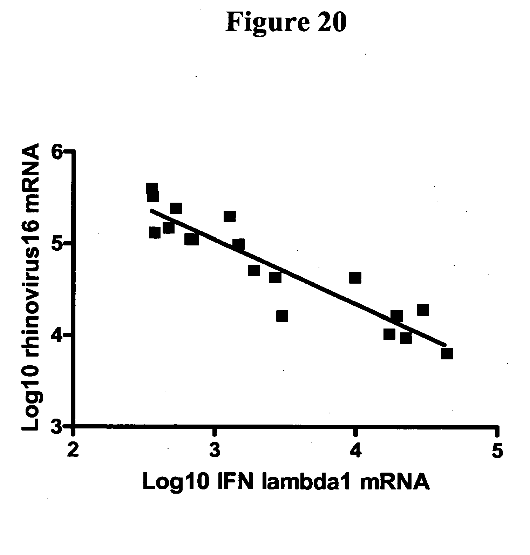

**Figure 20****.** IFNλ mRNA expression in primary human bronchial epithelial cells is strongly related to virus load. The greater the IFNλ expression, the less virus replication occurred. This data indicates that IFNλ is associated with anti-viral activity in primary human bronchial epithelial cells.

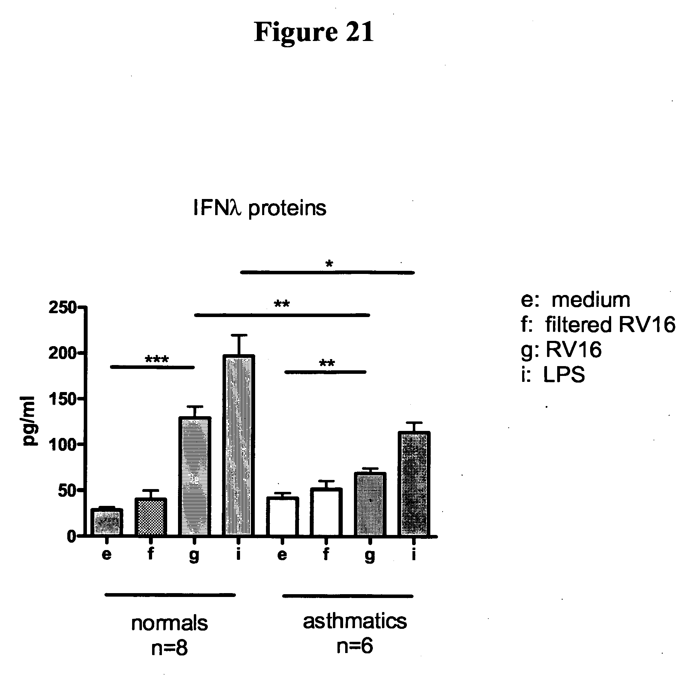

**Figure 21****.** IFNλ production in response to both rhinovirus infection and LPS stimulation of bronchoalveolar lavage cell pellets is deficient in asthmatics relative to normal individuals. Normally, more than 80% of bronchoalveolar lavage cells are macrophages. This data indicates that asthmatics are deficient in terms of IFNλ production from macrophages as well as the previous data from the epithelial cells.

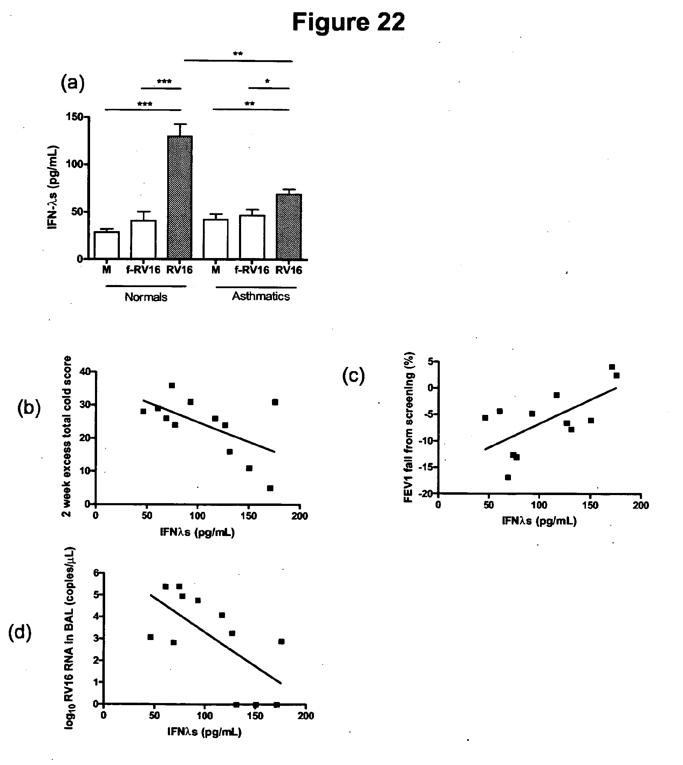

**Figure 22****: IFNλ production from bronchoalveolar cell supernatants stimulated *ex vivo* with rhinovirus.** IFNλ production in response to rhinovirus infection of bronchoalveolar lavage cell pellets is strongly related to severity of clinical colds, severity of reductions in lung function and to virus load in asthmatic and normal subjects experimentally infected with rhinovirus in vivo. In these experiements IFNλ production from bronchoalveolar lavage cell pellets in response to rhinovirus infection in vitro was determined at baseline, and subjects were then infected with rhinovirus in vivo 2 weeks later. During this in vivo infection, cold symptoms, reductions in lung function and lower airway virus load were all assessed to monitor severity of clinical illness during an in vivo infection. IFNλ production at baseline was strongly related to severity of colds, severity of asthma exacerbation as determined by reductions in lung function, and to bronchoalveolaor lavage virus load during the in vivo infection. These data clearly indicate that IFNλ production is a major determinant of severity of clinical illness during respiratory virus infections in vivo, and indicate that IFNλ administration should reduce symptoms, virus load and severity of reductions in lung function during respiratory virus infection in asthmatic subjects.
**Figure 23****: Time course of induction of type I and type III interferons in response to rhinovirus 16 infection in BEAS-2B cells.**
   **(a)** The mRNA expression of different alpha interferon subtypes was studied by Taqman PCR. For detection of various alpha interferon subtypes two pairs of Taqman PCR primers and probes were selected. First primer and probe set detects subtypes 1,6 and 13, second primer and probe set detects subtypes 4,5,8,10,14,17,21. The expression of type 1 interferons was detected by first primer pair -IFNA.1, but no significant induction of mRNA expression of these type 1 interferons by rhinovirus 16 was found. Using the second primer pair - IFNA.2 a statistically significant increase of type 1 interferon mRNA expression in comparison to medium was observed, but only at 8 hours from infection (p<0.001).
   **(b).** The expression of IL-29 and beta interferon mRNA was studied by Taqman PCR in the same experiments. The induction of IL-29 mRNA expression by rhinovirus 16 was statistically significant increased at 8 hour time point and we detected even higher induction by 24 hours (p<0.001). IFN beta mRNA expression was also induced by rhinovirus 16 at 24 hours (p<0.001). Thus RFNλ mRNA production occurred earlier than beta, was more sustained than alpha and was induced to a greater degree than either alpha or beta IFN production.
   **(c).** The production of interferon-beta was measured by ELISA in the same experiments. By 24 hours statistically significant induction of interferon-beta protein was detected in rhinovirus 16 infected BEAS-2B cells (p<0.01).
   **(d).** The production of IL-29 was measured by ELISA in the same experiments. By 24 hours statistically significant induction of IL-29 protein was detected in rhinovirus 16 infected BEAS-2B cells (p<0.001). Thus consistent with the mRNA data, production of IFNλ protein was 5 fold greater than production of beta IFN. Alpha IFN proteins were undetectable in these experiments.

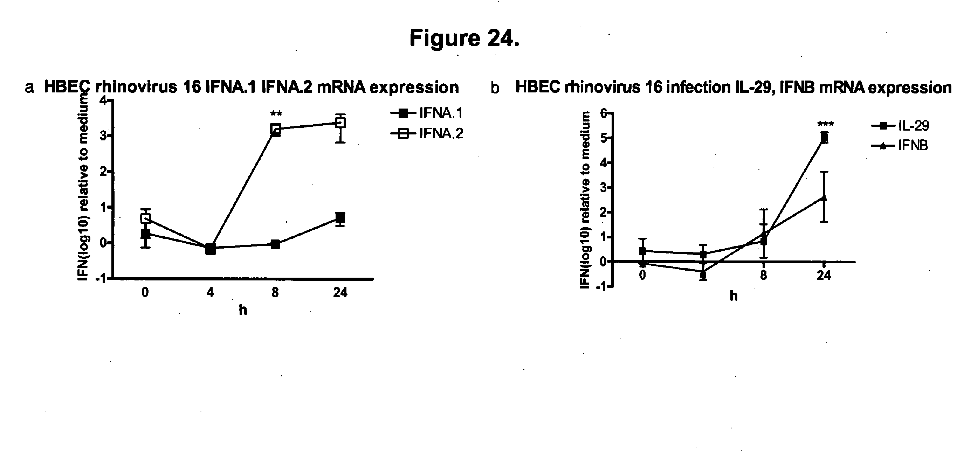

**Figure 24****: Time course of IFN production in response to rhinovirus 16 infection in primary human bronchial epithelial cells**
   **(a)** The mRNA expression of different alpha interferon types was assessed during a rhinovirus 16 time course at 0, 4, 8 and 24-hour time points in human bronchial epithelial cells cells by Taqman PCR. Alpha-interferons detetected by IFNA.1 primer pair were not induced by rhinovirus 16 while those detected by the IFNA.2 were significantly induced (p<0.01) over medium at 8 hours but at 24 hours there was no statistically significant induction.
   **(b).** The expression of IL-29 and beta interferon mRNA in human bronchial epithelial cells infected by rhinovirus 16 was studied by Taqman PCR in the same experiements. IL-29 mRNA expression was significantly induced at 24 hours (p<0.001). Interferon-beta demonstrated no significant induction at any time point.

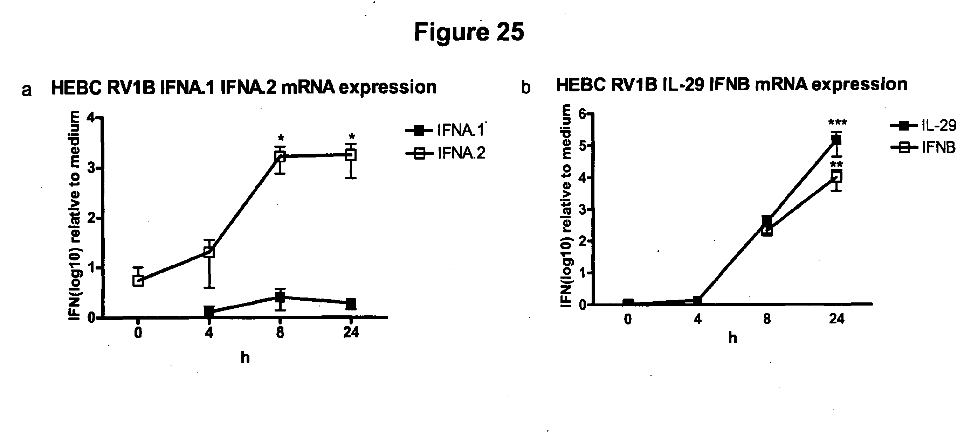

**Figure 25****. Time course of IFN production in response to rhinovirus 1B infection in human bronchial epithelial cells**
   IFN alpha, IFN beta and IL-29 mRNA expression was also assessed during a rhinovirus 1B time course at 0, 4, 8 and 24-hour time points by Taqman PCR.
   **(a)** Alpha-interferons detetected by IFNA.1 primer pair were not induced by rhinovirus 16. mRNA of alpha-interferons detected by second primer pair IFNA.2 were induced by rhinovirus 1B at 8 and 24 hours (p<0.05).
   **(b).** With IL-29 a very high level of induction (2 logs greater than those detected by the IFNA.2 primer pair) was detected at 24 hours (p<0.001). Induction of interferon-beta mRNA was also detected at 24 hours (p<0.01), though this induction was 1 log less than that observed for IL-29.

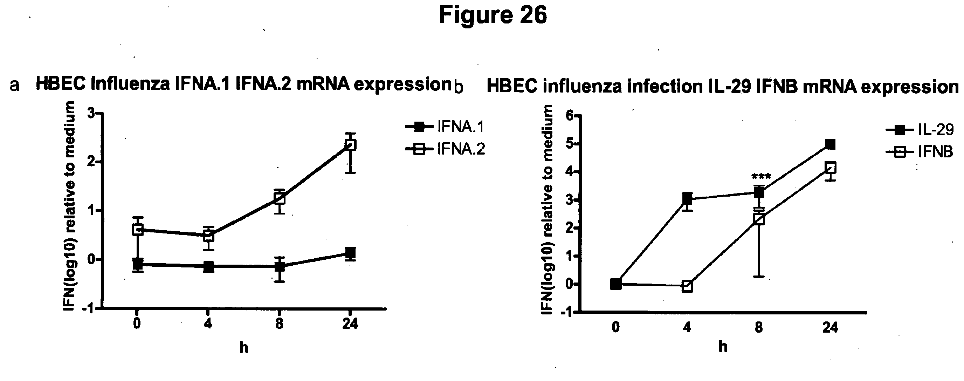

**Figure 26****. Time course of IFN production in response to influenza virus infection in human bronchial epithelial cells**
   (a) The expression of different alpha interferon types mRNA was assessed during a influenza virus time course at 0, 4, 8 and 24-hour time points in human bronchial epithelial cells by Taqman PCR. Neither alpha-interferons detected by the IFNA.1 primer pair nor those detected by the IFNA.2 primer pair were significantly induced by influenza virus at any time point.
   **(b).** The expression of IL-29 and beta-interferon mRNA was studied in human bronchial epithelial cells infected by influenza virus by Taqman PCR in the same experiements. IL-29 was significantly induced by influenza virus at 8 hours after infection (p<0.001) and increased further at 24hrs. Interferon-beta mRNA induction was not significantly induced at any time point, though increases were observed at 8 and 24hrs. IL-29 mRNA expression was greater than that of alpha and beta IFNs at all time points.

### Example 1: Rhinovirus induces IFN-λs in bronchial epithelial cells.

Aim: Investigate whether RV induces IFN-λs *in vitro* and whether IFN-λs induce antiviral activity against RV infection in bronchial epithelial cells.

Outline of methods: The human bronchial epithelial cell-line BEAS2B was infected with RV. TaqMan® PCR was used to identify IFN-λs mRNA expression and a bioassay was employed for corresponding protein production. BEAS2B cells were treated for 24h with different doses of IFN-λ1 before infection with RV. Both TaqMan® PCR for viral RNA in cell lysates and viral titration of the BEAS2B supernatant were performed to investigate the antiviral effect.

Results: IFN-λ1, IFN-λ2 and IFN-λ3 mRNA was increased after 4h (p<0.05) and peaked at 8h post-infection (p<0.001). The increase was demonstrated to be dose-responsive to RV-16 at 24h post-infection (p<0.001). Infection with RV-9 and RV-1B demonstrated that the response was serotype and receptor independent. UV-inactivation of RV-16 completely inhibited the up-regulation, indicating that active viral replication is required. EMSA assay detected the presence of IFN-λs proteins in the supernatant of BEAS2B 24h after the infection. Finally both TaqMan® PCR for viral RNA in cell lysates (p<0.001) and viral titration (p<0.001) showed a dose-dependent anti-viral effect of IFN-λ1 to RV16 infection.

Conclusions: This study demonstrated that RV infection of bronchial epithelial cell-line leads to the production of IFN-λs and that these proteins may play an important role in the antiviral response to RV.

### Example 2: Viral infection in asthma exacerbations: role of interferon lambda.

Aim: Investigate whether RV16 induces IFN-λs and if this production is associated with increased susceptibility to rhinovirus infections in asthmatics.

Methods: The human bronchial epithelial cell-line BEAS2B and bronchial primary cells from asthmatics (6) and normal patients (5) were infected with RV16. TaqMan® PCR for IFN-λ mRNA expression was used. BEAS2B cells were treated with IFN-λ1 before infection with RV16. Both TaqMan® PCR for viral RNA in cell lysates and viral titration of the BEAS2B supernatant were performed to investigate the antiviral effect. TaqMan® PCR for viral RNA in cell lysates of primary cells was used also to test weather RV16 infectivity was different between primary cells from asthmatics and normals.

IFN-λs in increasing susceptibility to rhinovirus infections in asthmatics. Results: IFN-λs mRNA was increased both in BEAS2B and in primary cells with the peak at 8h infection. In BEAS2B both TaqMan® PCR for viral RNA and viral titration showed a dose-dependent anti-viral effect of IFN-λ1 to RV16 infection. Primary bronchial epithelial cells produced significantly lower amount of IFN-λ after RV16 infection (p<0.05) as compared to normal controls. Conversely RV16 replication was higher (p<0.05) in bronchial epithelial cells from asthmatic subjects.

Conclusions: RV16 infection of bronchial epithelial cells leads to the production of IFN-λs. This production is deficient in asthmatic subjects and may thus be a factor in increasing susceptibility to rhinovirus infections in asthmatics.

### Further Materials and methods

### Obtaining of primary bronchial epithelial cells

All subjects were nonsmokers, with no exacerbations or respiratory tract infections in the preceding 4 wk. Allergy skin tests used a panel of common aeroallergens and were considered positive if the wheal response was >3 mm than the negative control. Lung function was assessed by spirometry and bronchial hyperresponsiveness by histamine challenge. Asthma was diagnosed in atopic individuals with a consistent history and evidence of bronchial hyperresponsiveness (defined by a PC₂₀ histamine <8 mg/ml) and was categorized in accordance with the GINA guidelines (National Heart, Lung and Blood Institute. 1995. Global Strategy for Asthma Management and Prevention. 96-369). Healthy controls had no previous history of lung disease, normal lung function, no evidence of bronchial hyperresponsiveness, and were nonatopic. The study was approved by the Southampton University Hospital Ethics Committee. All subjects gave written informed consent.

### Bronchial epithelial cell tissue culture

Primary BECs were grown from bronchial brushings (>95% epithelial cells), which were obtained by fiber-optic bronchoscopy in accordance with standard guidelines (Hurd, S.Z. 1991. J. Allergy Clin. Immunol. 88:808-814); there was no significant difference in the proportion of columnar and basal cells isolated from normal or asthmatic donors. Cell culture and characterization was performed as described previously (Bucchieri, F., J. Lordon, A. Richter, D. Buchanan, R. Djukanovic, S.T. Holgate, and D.E. Davies. 2001. Am. J. Respir. Cell Mol. Biol. 27:179-185; Lordan, J.L., F. Bucchieri, A. Richter, A. Konstantinidis, J.W. Holloway, M. Thornber, S.M. Puddicombe, D. Buchanan, S.J. Wilson, R. Djukanovic, et al. 2002. J. Immunol. 169:407-414). The cultured cells were all cytokeratin positive and exhibited a basal cell phenotype, as evidenced by the expression of cytokeratin 13, irrespective of the type of donor of the original brushings. Primary cultures were established by seeding freshly brushed BECs into hormonally supplemented bronchial epithelial growth medium (Clonetics) containing 50 U/ml penicillin and 50 µg/ml streptomycin. At passage two, cells were seeded onto 12-well trays and cultured until 80% confluent (Bucchieri *et al supra*) before exposure to RV-16.

### Generation and titration of RV

RV-16 stocks were generated and titrated from infected cultures of Ohio HeLa cells as described previously (Papi, A., and S.L. Johnston. 1999 J. Biol. Chem. 274:9707-9720). Cells were infected at a multiplicity of infection of 2. Confirmation of infection and quantification of viral production was assessed by HeLa titration assay (Papi, A., and S.L. Johnston, *supra*) and reverse transcription quantitative polymerase chain reaction (RT-qPCR), as described below. As negative controls, cells were treated with medium alone andUV inactivated RV-16 (Papi, A., and S.L. Johnston, *supra*).

### RT-qPCR and ELISA

RT-qPCR analysis of IFNλ mRNA and RV-16 viral RNA (vRNA) gene expression was performed on DNase treated RNA extracted from BECs using TRIzol (Life Technologies). Total RNA (1 µg) was reverse transcribed using avian myeloblastosis virus transcriptase (Promega) and random hexamers for IFNλ mRNA and 18S rRNA analysis or oligo (dT)₁₅ for RV-16 vRNA. Real-time detection used an iCyclerIQ detection system using a PCR protocol as follows: 42 cycles at 95°C for 15 s, 60°C for 1 min and 72°C for 15 s. IFNλ signals were normalized to 18S rRNA and relative quantification performed using the ΔΔCT method. Comparisons were made 8 h after infection. Quantification of RV-16 was achieved using a TAQman assay located in the 5' UTR in conjunction with the standard curve method. The standard curve was constructed using 10-fold serial dilutions of RV-16 5' NTR cDNA cloned into PCR 2.1 TOPO (Invitrogen). Relative values for RV detection were calculated by normalizing to the starting cell number. Probe: FAM/TAMRA 6-FAMTGAGTCCTCCGGCCCCTGAATG, forward primer (RVTM-1) 5'-GTGAAGAGCCSCRTGTGCT-3', reverse primer (RVTM-2) 5'-GCTSCAGGG-TTAAGGTTAGCC-3'.

### Statistical analysis

When data were normally distributed the mean and SD have been used, differences between groups have been analyzed using Student's *t* test, when not normally distributed data were analyzed using nonparametric equivalents and summarized using the median and IQR, multiple comparisons were first analyzed by the Kruskal Wallis test and then by individual testing if significant. Correlations were analyzed by Spearman's test. A p-value of <0.05 was considered significant.

### Diagnosis of asthma and COPD

### (i) Diagnosing COPD

The following information was taken from a publication titled: "Pocket guide to COPD diagnosis, management and prevention: a guide for heath care professionals", which is available from www.goldcopd.com.

A diagnosis of COPD should be considered in any individual who presents characteristic symptoms and a history of exposure to risk factors for the disease, especially cigarette smoking.

### Key Indicators for Considering a COPD Diagnosis

- Chronic cough: Present intermittently or every day. Often present throughout the day; seldom only nocturnal.
- Chronic sputum production: Any pattern of chronic sputum production may indicate COPD.
- Acute bronchitis: Repeated episodes.
- Dyspnea that is: Progressive (worsens over time). Persistent (present every day). Worse on exercise. Worse during respiratory infections.
- History of exposure to risk factors: Tobacco smoke (including popular local preparations). Occupational dusts and chemicals. Smoke from home cooking and heating fuel.

The diagnosis should be confirmed by spirometry. Where spirometry is unavailable, the diagnosis of COPD should be made using all available tools. Clinical symptoms and signs (abnormal shortness of breath and increased forced expiratory time) can be used to help with the diagnosis. A low peak flow is consistent with COPD but has poor specificity since it can be caused by other lung diseases and by poor performance. In the interest of improving the accuracy of a diagnosis of COPD, every effort should be made to provide access to standardized spirometry.

When performing spirometry, measure:
- Forced Vital Capacity (FVC) and
- Forced Expiratory Volume in one second (FEV1).

Calculate the FEV1/FVC ratio. Spirometric results are expressed as % Predicted using appropriate normal values for the person's sex, age, and height.

Patients with COPD typically show a decrease in both FEV1 and FEV1/FVC. The degree of spirometric abnormality generally reflects the severity of COPD. However, both symptoms and spirometry should be considered when developing an individualized management strategy for each patient.

### Classification of COPD by Severity

Stage 0: At Risk - Chronic cough and sputum production; lung function is still normal.

Stage I: Mild COPD - Mild airflow limitation (FEV1/FVC < 70% but FEV1 ≥ 80% predicted) and usually, but not always, chronic cough and sputum production.
- At this stage, the individual may not be aware that his or her lung function is abnormal.

Stage II: Moderate COPD - Worsening airflow limitation (50% ≤ FEV1 < 80% predicted), and usually the progression of symptoms, with shortness of breath typically developing on exertion.

Stage III: Severe COPD - Further worsening of airflow limitation (30% ≤ FEV1 < 50% predicted), increased shortness of breath, and repeated exacerbations which have an impact on patients' quality of life.
- Exacerbations of symptoms, which have an impact on a patient's quality of life and prognosis, are especially seen in patients with FEV1< 50% predicted.

Stage IV: Very Severe COPD - Severe airflow limitation (FEV1 < 30% predicted) or FEV1 < 50% predicted plus chronic respiratory failure. Patients may have very severe (Stage IV) COPD even if the FEV1 is > 30% predicted, whenever these complications are present.
- At this stage, quality of life is very appreciably impaired and exacerbations may be life-threatening.

### Differential Diagnosis

A major differential diagnosis is asthma. In some patients with chronic asthma, a clear distinction from COPD is not possible using current imaging and physiological testing techniques. In these patients, current management is similar to that of asthma. Other potential diagnoses are usually easier to distinguish from COPD:

Below are listed suggestive features that may be used to distinguish a number of different disorders. These features tend to be characteristic of the respective diseases, but do not occur in every case. For example, a person who has never smoked may develop COPD (especially in the developing world, where other risk factors may be more important than cigarette smoking); asthma may develop in adult and even elderly patients.

### Differential Diagnosis of COPD

| | |
|---|---|
| COPD: | Onset in mid-life. Symptoms slowly progressive. |
| | Long smoking history. |
| | Dyspnea during exercise. |
| | Largely irreversible airflow limitation. |
| Asthma: | Onset early in life (often childhood). |
| | Symptoms vary from day to day. |
| | Symptoms at night/early morning. |
| | Allergy, rhinitis, and/or eczema also present. |
| | Family history of asthma. |
| | Largely reversible airflow limitation. |
| Congestive Heart | |
| Failure: | Fine basilar crackles on auscultation. |
| | Chest X-ray shows dilated heart, pulmonary edema. |
| | Pulmonary function tests indicate volume restriction, not airflow limitation. |
| Bronchiectasis: | Large volumes of purulent sputum. |
| | Commonly associated with bacterial infection. |
| | Coarse crackles/clubbing on auscultation. |
| | Chest X-ray/CT shows bronchial dilation, bronchial wall thickening. |
| Tuberculosis: | Onset all ages. |
| | Chest X-ray shows lung infiltrate or nodular lesions. |
| | Microbiological confirmation. |
| | High local prevalence of tuberculosis. |

### (ii) Diagnosing asthma

The following information was taken from a publication titled: "Pocket guide for asthma prevention and management", which is available from www.ginasthma.com.

Asthma can often be diagnosed on the basis of symptoms. However, measurements of lung function, and particularly the reversibility of lung function abnormalities, greatly enhance diagnostic confidence.

### Is It Asthma?

Consider asthma *if any* of the following signs or symptoms are present.
- Wheezing-high-pitched whistling sounds when breathing out-especially in children. (A normal chest examination does not exclude asthma.)
- History of any of the following:
   Cough, worse particularly at night
   Recurrent wheeze
   Recurrent difficult breathing
   Recurrent chest tightness.
      (Note: Eczema, hay fever or a family history of asthma or atopic diseases are often associated with asthma.)
- Symptoms occur or worsen at night, awakening the patient.
- Symptoms occur or worsen in the presence of:
   Animals with fur
   Exercise
   Aerosol chemicals
   Pollen
   Changes in temperature
   Respiratory (viral) infections
   Domestic dust mites
   Smoke
   Drugs (aspirin, beta blockers)]
   Strong emotional expression
- Reversible and variable airflow limitation-as measured by using a spirometer (FEV1 and FVC) or a peak expiratory flow (PEF) meter. When using a peak flow meter, consider asthma if:
   - PEF increases more than 15 percent 15 to 20 minutes after inhalation of a
      rapid-acting_2-agonist, *or*
   - PEF varies more than 20 percent from morning measurement upon arising to measurement 12 hours later in patients taking a bronchodilator (more than 10 percent in patients who are not taking a bronchodilator), or
   - PEF decreases more than 15 percent after 6 minutes of sustained running or exercise.

### Peak Flow Meters: Uses and Technique

- Lung function measurements assess airflow limitation and help diagnose and monitor the course of asthma.
- To assess the level of airflow limitation, two methods are used. Peak flow meters measure peak expiratory flow (PEF), and spirometers measure forced expiratory volume in 1 second (FEV1) and its accompanying forced vital capacity (FVC). The accuracy of all lung function measurements depend on patient effort and correct technique.
- Several kinds of peak flow meters and spirometers are available, and the technique for use is similar for all. To use a peak flow meter:
   - Stand up and hold the peak flow meter without restricting movement of the marker. Make sure the marker is at the bottom of the scale.
   - Take a deep breath, put the peak flow meter in your mouth, seal your lips around the mouthpiece, and breathe out as hard and fast as possible. Do not put your tongue inside the mouthpiece.
   - Record the result. Return the marker to zero.
   - Repeat twice more. Choose the highest of the three readings.
- Daily PEF monitoring for 2 to 3 weeks is useful, when it is available, for establishing a diagnosis and treatment. If during 2 to 3 weeks a patient cannot achieve 80 percent of predicted PEF (predicted values are provided with all peak flow meters), it may be necessary to determine a patient's personal best value, e.g. by a course of oral glucocorticosteroid.
- Long-term PEF monitoring is useful, along with review of symptoms, for evaluating a patient's response to therapy. PEF monitoring can also help detect early signs of worsening before symptoms occur.

Note: Examples of available peak flow meters and instructions for use of inhalers and spacers can be found on www.ginasthma.org.

### Diagnostic challenges include the following:

- Young children whose primary symptom is recurrent or persistent cough or who wheeze with respiratory infections are often misdiagnosed as having bronchitis or pneumonia (including acute respiratory infection -ARI) and thus ineffectively treated with antibiotics or cough suppressants. Treatment with asthma medication can be beneficial and diagnostic.
- Many infants and young children who wheeze with viral respiratory infections may not develop asthma that persists through childhood. But they may benefit from asthma medications for their wheezing episodes. There is no certain way to predict which children will have persistent asthma, but allergy, a family history of allergy or asthma, and perinatal exposure to passive smoke and allergens are more strongly associated with continuing asthma.
- Asthma should be considered if the patient's colds repeatedly "go to the chest" or take more than 10 days to clear up, or if the patient improves when asthma medication is given.
- Tobacco smokers and elderly patients frequently suffer from chronic obstructive pulmonary disease (COPD) with symptoms similar to asthma. Yet they may also have asthma and benefit from treatment. Improvement in PEF after asthma treatment is diagnostic.
- Workers who are exposed to inhalant chemicals or allergens in the workplace can develop asthma and may be misdiagnosed as having chronic bronchitis or chronic obstructive pulmonary disease. Early recognition (PEF measurements at work and home), strict avoidance of further exposure, and early treatment are essential.
- Asthma attacks may be difficult to diagnose. For example, acute shortness of breath, chest tightness and wheezing can also be caused by croup, bronchitis, heart attacks, and vocal cord dysfunction. Using spirometry, establishing reversibility of symptoms with bronchodilators, and assessing the history of the attack (e.g. whether it was related to exposures that commonly make asthma worse) aid the diagnosis. A chest x-ray can help rule out infection, large airway lesions, congestive heart failure, or aspiration of a foreign object.

**Example 3: Expression of Alpha-interferons, Beta-interferons and Lambda-interferons in Epithelial Cells and PBMCs after Respiratory Virus Infections.**

In this study, the potential of different cell types such as BEAS-2B, human bronchial epithelial cells (HBEC) and PBMC (as a model for macrophages) to express and produce various type 1 and type III interferons upon respiratory virus infection was investigated. Sets of primers and probes were designed for quantitive PCR of various type 1 and type III interferons. In BEAS-2B cells induction of IFN-α mRNA expression was detected by 8 hours from 0-time point, induction of IL-29 mRNA from 0-time point was detected by 8-hours with peak at 24 hours and induction of IFN-β from 0-time point was detected by 24 hours. By ELISA we also observed production of IL-29 and IFN-β protein by 24 hours. In HBEC induction of IFNA mRNA expression was detected by 8 hours from 0-time point and induction of IL-29 mRNA from 0-time point by 24 hours. In PBMC induction of IFNA, IL-29 and IFNB mRNA expression by 8 hours from 0-time point were demonstrated. Induction of IFN-α, IFN-β and IL-29 protein by ELISA was additionally shown.

### Additional information on rhinoviruses

Rhinoviruses are small RNA viruses. They belong to picornaviridae family. More than 100 serotypes of rhinoviruses have been identified. According to the type of the receptor for binding rhinoviruses are divided into two groups. Major group approximately 90% of all RV serotypes use ICAM-1 molecule and minor group approximately 10% of all RV serotypes use low density lipoprotein receptor (N.G. Papadopoulos, S.L.Johnston: Rhinoviruses. Principles and practice of clinical virology. 5th edition2004, 361-377).

Recent work indicates that asthmatic individuals are more susceptible to naturally occurring rhinovirus (RV) infection than normal individuals in that lower respiratory tract symptoms and changes in PEF were more severe and of longer (Come et al., Lancet (2002) 359, 831-834).So the important question is what differences occur in lower airway of asthmatics in comparison to normal subjects during RV infection and lead to asthma exacerbation.

It was demonstrated that in asthmatics RV induces greater severity of lower respiratory symptoms which is accompanied by higher concentrations of inflammatory cells: lymphocytes, NK cells, eosinophils and neutophils in BALRV infection induces inflammatory response (IL-6, IL-8, RANTES, IL-16 and upregulation of ICAM-1) in bronchial epithelium (N.G. Papadopoulos, P.J. Bates, P.G. Bardin et al. J Infect Dis 181 (2000), pp. 1875-1884; S.L. Johnston, A. Papi, P.J. Bates, J.G. Mastronarde, M.M. Monick and G.W. Hunninghake,. J Immunol 160 (1998), pp. 6172-6181). PBMCs from asthmatics exposed to RV *ex vivo* demonstrated decreased levels of type I cytokines and increased levels of type 2 cytokines when compared to normals (Papadopoulos et al. Thorax 57 (2002)). Moreoover, as already noted above, more recently primary epithelial bronchial cells from asthmatics exposed to RV *ex vivo* were observed to demonstrate decreased levels of IFN-β when compared to normals (Wark et al J.Exp. Med. (21 st March 2005) 201, 937-947)

As also previously discussed above, Type 1 interferons such as IFN-α, IFN-β and the more recently discovered type III interferons (IFN-λs) play a vital role in innate immune response against viruses. They induce lots of IFN-inducible genes with antiviral properties and as it has been shown recently induce apoptosis in virally infected cells (Takaoka A, Hayakawa S, Yanai H, et al. Nature 2003; 424(6948):516-523).

As there is no small animal model for rhinovirus infection, it is very important to use proper cell cultures which are being infected by rhinoviruses. It is known that rhinovirus infects and replicates in respiratory epithelial cells of lower respiratory tract (N.G. Papadopoulos et al J Med Virol 58 (1999), pp. 100-104). As it is not much known about the induction of type 1 and type III interferons in epithelial cells, in this study we tried to show how different cell types such as primary bronchial epithelial cells, BEAS-2B and PBMC (as a model for macrophages) express and produce various type 1 and type III interferons upon different respiratory virus infection.

### MATERIALS AND METHODS

### Human bronchial epithelial cell tissue culture

Human bronchial epithelial cells (HBECs) were purchased from Cambrex, USA. Primary cultures were established by seeding bronchial epithelial cells into hormonally supplemented bronchial epithelial growth medium (BEBM; Cambrex, USA) containing 2ml BPE, 0.5ml insulin, HC 0.5 ml, GA-1000 0,5 ml, retinoic acid 0.5 ml, transferrin 0.5 ml, triiodothyronine 0.5 ml, epinephrine 0.5 ml, hEGF 0.5 ml (Cambrex, USA). At passage 1 cells were seeded onto 12 well trays and cultured until 80% confluent (Bucchieri et al., Asthamatic bronchil epithelium is more susceptible to oxidant-induced apoptoisis. Am. J. Respir. Cell Mol. Biol. 27, 179) before exposure to RV-16, RV-1B and influenza virus.

### Cell and viral culture

The human bronchial epithelial cell line BEAS-2B were cultured in RPMI-1640 supplemented with 10% FCS (Invitrogen). RV serotypes 16 and 1B were grown in HeLa cells and prepared as previously described (Papi and Johnston (1999) Rhinovirus infection induces expression of its own receptor intercellular adhesion molecule 1 (ICAM-1) via increased NF-kB- mediated transcription J. Biol. Chem. 274, 9707-9720) Viruses were titrated on HeLa cells to ascertain their TCID₅₀/ml (Johnston and Tyrell (1995) Rhinoviruses, p. 253-263 In Diagnostic procedures for viral, rickettsial and Chlamydial infections, ed Lennette and Schmidt, American Public helath Association, Washington, D. C.). The identities of all RVs were confirmed by titration on HeLa cells and neutralisation using serotype-specific antibodies. UV inactivation was performed as previously described (Johnston et al. (1998) Low grade rhinovirus infection indcuces a prolonged release of IL-8 in pulmonary epithelium. J. Immunol. 160, 6172-6181) and filtered virus was produced by passing RV stocks through a 30 KDa membrane (Millipore) at 10 000g for 5 min.

### Infection of cells with RV

BEAS-2B cells were cultured in 12-well tissue culture plates (Nalge Nunc) for 24hours before being placed into 2% FCS RPMI medium for a further 24-hours. Cells were infected with RV for 1-hour with shaking at room temperature, before the virus was removed and replaced with 1ml of 2% FCS RPMI medium. Cells supernatants and RNA lysates were harvested at the times indicated. Supernatants and lysates were stored at -80°C until required.

### PBMC separation and rhinovirus 16 infection

PBMCs were separated from whole blood using gradient density centrifugations (Sigma). 4x10(6) cells/2ml were exposed to rhinovirus 16 for 1 hour. At the end of exposure time cells were washed and medium was changed.

### RNA extraction, reverse transcription and TaqMan® real-time PCR

RNA was extracted from cells using the RNeasy method following the manufacturers instructions, including the optional DNaseI digestion of contaminating DNA (Qiagen). CDNA was synthesised using Omniscript RT and components as directed by the manufacturer (Qiagen).

Primers were purchased from Invitrogen and probes from Qiagen. TaqMan® analysis of alpha-interpherons, IL-29 and IFNB mRNA was normalised with respect to 18s rRNA. For detecting of alpha-interferons types 1,6 and 13 IFNα.1 set of primers and probe was used ( IFNA.1 forward -5'-CAG AGT CAC CCA TCT CAG CA -3, IFNA.1 reverse- 5'-CAC CAC CAG GAC CAT CAG TA-3' and 5'-FAM-TAMRA labelled probe - 5'-ATC TGC AAT ATC TAC GAT GGC CTC gCC-3'). For detecting of alpha-interferons types 2,4,5,8,10,14,17,21 1 IFNa.2 set of primers and probe was used (IFNa.2 forward -5'-CTG GCA CAA ATG GGA AGA AT -3', IFNA.2 reverse- 5'- CTT GAG CCT TCT GGA ACT GG -3' and 5'-FAM-TAMRA labelled probe - 5'- TTT CTC CTG CCT GAA GGA CAG ACA Tga-3'. For IL-29 detection we used forward primer - 5'GGA CGC CTT GGA AGA GTC ACT'3, reverse - 5'-AGA AGC CTC AGG TCC CΛΛ TTC'-3 and 5'-FAM-TAMRA labelled probe - 5'- AGT TGC AGC TCT CCT GTC TTC CCC G-3'. For interferon-beta detection we used forward primer - 5'-CGC CGC ATT GAC CAT CTA-3', reverse - 5'-GAC ATT AGC CAG GAG GTT CTC A-3' and 5'-FAM-TAMRA labelled probe - 5'-TCA GAC AAG ATT CAT CTA GCA CTG GCT GGA-3'. For 18s, each reaction contained 18STM. (CGC CGC TAG AGG TGA AAT TCT), 18STM.2 (CAT TCT TGG CΛΛ ΛTG CTT TCG), 5'-FAM-TAMRA labelled probe (5'-ACC GGC GCA AGA CGG ACC AGA) and 2µl cDNA diluted 1/100 in 1x Quantitect Probe PCR Master Mix (Qiagen). The reactions were analysed using an ABI7000 Automated TaqMan (Applied Biosystems). The amplification cycle consisted of 50°C for 2 minutes, 94°C for 10 minutes and 40 cycles of 94°C for 15 seconds, 60°C for 15 seconds.

*Enzyme-Linked Immunosorbent Assay to Evaluate IFN-A, IL-29 and IFNB release* Interferon-alpha, interferon-beta and IL-29 proteins were quantified by ELISA in supernatants from untreated and infected cell cultures collected and stored at - 80°C using commercially available paired antibodies and standards, following the manufacturers instructions. High Sensitivity Interferon-alpha Human Biotrak ELISA System by Amersham Biosciences for interferon-alpha. Human Interferon-beta ELISA kit was purchased from Fujirebio Inc. All the measurements were done according to manufactures' instructions. The detection limits for described assays are 0.63 pg/ml for interferon-alpha, 2.5 UI/ml for interferon-beta and 0.01 for IL-29.

### Quantitative ELISA for IFNλs

ELISA 96 well plates (Nunc Maxisorp) were coated with detecting antibody (100 µl per well of Monoclonal Anti-human IL-29/IFN-λ1 Antibody diluted in PBS from R&D system catalogue number MAB 15981 at concentration of 1 µg/ml) and left at room temperature overnight.. The next morning plates were washed twice in PBS with 0.1% of Tween 20 and than blocked at room temperature with 220 µl per well of a solution of 2% BSA. After 2 hours plates were washed twice and 100 µl of undiluted samples and 100 µl of standard samples were added in the wells. Samples and standard were both tested in duplicate. Standard was set up in diluent buffer (PBS with 1% BSA and 0.1% Tween 20) using Recombinant Human IL-29/IFN-λ1 from R&D system starting from 3 ng/ml down to approximately 10 pg/ml. 100 mcl of diluent buffer were added in same wells as negative controls. After 2 h plates were washed twice and 100 µl of secondary antibody were added (Anti-human IL-29/IFN-λ1 Antibody from R&D system catalog numeber AF1598 reconstituted in PBS and diluted in diluent buffer at a concentration of 1 µg/ml). According to manufacture instruction these antibodies have respectly 5% for the monoclonal and 25% for the polyclonal cross-reactivity with IFN-λ2 and IFN-λ3. After 2 hours plates were washed and 100 µl of biotinylated antidody from AutogenBioclear catalog number ABN022B diluted 1 in 5000 in diluent buffer were added to each well for 2 hours. Plates were washed twice and 100 µl of streptavidene-HRP conjugated diluted 1 in 5000 in diluent buffer were added in each well for 15 minutes. Plates were washed tree times and 100 µl of TMB substrate solution were added and the reaction was stopped with 50 µl of 1.8 M oh H₂SO₄ solution.

### Statistical analysis

Data are presented as mean ± SEM. All data were analysed using one-way ANOVA and Bonferroni's multiple comparison *post hoc* test. Data were accepted as significantly different when *p*<0.05.

### RESULTS

*Time course of type 1 and type III interferons mRNA expression in BEAS-2B cells* The expression of type 1 and type III interferons was studied during time course infection of BEAS-2B cells with RV 16 by Taqman PCR. For detection of various alpha- interferon subtypes, two pairs of Taqman PCR primers and probes were selected. First primer and probe set detects subtypes 1,6 and 13, second primer and probe set detects subtypes 4,5,8,10,14,17,21. Primer and probe sets for detection of IL-29 (IFN-λ) and interferon-beta were also designed. Figure 23a demonstrates the expression of type 1 interferons detected by IFNα.1, but no significant induction of these type 1 interferons mRNA by rhinovirus 16. With IFNα.2 statistically significant increase of type1 interferon expression occurred in comparison to medium by 8 hours compared to 0-hour time point. At 0 4 and 24 time points no induction was found. IL-29 mRNA expression was also statistically significant increased at 8 hour time point and we detected even higher induction by 24 hours (p<0.05) figure 23b.Interferon-beta mRNA expression was induced by rhinovirus 16 just once by 24 hours (p<0.05). 1000 fold induction was detected over medium. All the results were statistically significant (p<0.05) from 0-hour time point.

### Detection of Interferon-alpha, interferon-beta and IL-29 proteins in RV infected BEAS-2B cells

No significant induction of interferon-alpha protein during rhinovirus 16 infection was detected in BEAS-2B cells. Only traces of IFNA protein from approximately 0.3 to 0.5 pg/lm were detected by ELISA with range of detection from 0.63 to 20 pg/ml. The induction of IFNβ protein production was observed by 24 hours (figure 23c). The level of IFNβ protein production was statistically significantly (p<0.05) increased in rhinovirus 16 infected BEAS-2B cells in comparison to non infected cells.BEAS-2B cells infected with rhinovirus 16 produced high level of IL-29 protein by 24 hours (p<0.05) (figure 23d).

### Time course of type 1 and type III interferon mRNA expression in Primary Bronchial Epithelial cell during rhinovirus 16 infection

IFNα, IFNβ and IL-29 mRNA expression was assessed during a rhinovirus 16 time course at 0, 4, 8 and 24-hour time points. Alpha-interferons detetected by IFNA.1 primer pair were expressed at all time points but never upregulated by rhinovirus 16. Using IFNA.2 we observed no induction by 4 hours and 10000 fold statistically significant induction over medium by 8 hours (p<0.05) which was still elevated by 24 hours (figure 24a). With IL-29 mRNA we observed slight induction by 4 and 8 hours and peak -1000000 fold induction over medium by 24 hours (p<0.05). Interferon-beta demonstrated no induction by 0, 4 and 8 hour time points, but was induced by rhinovirus 16 at 8 and 24 hours -100000 fold induction over medium (figure 24b).

### Time course of type 1 and type III interferon mRNA expression in Human Bronchial Epithelial cell during rhinovirus 1B infection

Alpha-interferons, interferon-beta and IL-29 mRNA expression was also observed during a rhinovirus 1B time course at 0, 4, 8 and 24-hour time points. Alpha-interferons detected by IFNα.1 primer pair were also expressed at all time points but not induced by rhinovirus 16. But mRNA of alpha-interferons detected by second primer pair IFNα.2 were not induced by rhinovirus 1B by 0 an 4 hours and peaked by 8 and 24 hours - 10000 fold induction over medium. These results reached the statistical significance (p<0.05) (figure 25a). IL-29 mRNA was not induced by rhinovirus 1B by 0 and 4 hours. But some induction was observed by 8 hours (1000 fold induction over medium) and very high level of induction was detected by 24 hours - 1000000 fold induction over medium (p<0.05).. No induction of interferon-beta was observed by 4 hours, 100 fold induction over medium by 8 hours (not statistically significant) and peak at 24 hours -100000 fold induction over medium (p<0.05) (figure 25b).

### Time course of type 1 and type III interferon mRNA expression in Human Bronchial Epithelial cell during influenza infection

IFNα, IFNβ and IL-29 mRNA expression was also observed during influenza virus time course at 0, 4, 8 and 24-hour time points. Alpha-interferons detetected by IFNA.1 primer pair were again expressed at all time points but not induced by influenza virus. Alpha-interferons detected by IFNα2 were not upregulated by 0 and 4 hours. Slight 10 fold induction was observed by 8 hours and 100 fold induction by 24 hours (figure 26a). IL-29 was induced by influenza virus at 4 and 8 hour time point (1000 fold induction over medium) and peaked at 24 hours (1000000 fold induction over medium).IFNβ m RNA induction was not seen at 4 and 8 hours. But 100 fold induction was detected at 8 hours and peaked at 24 hours- 10⁵ fold induction over medium (figure 26b).

### Detection of Interferon-alpha, interferon-beta and IL-29 protein in HBEC cells.

No production of Interferon-alpha, interferon-beta and IL-29 has been detected in HBEC cells infected with rhinovirus 16, 1 B and influenza virus.

### DISCUSSION

As already described the expression of alpha-interferon types detected by first primer pair (types 1,6 and 13) was detected in both epithelial cell cultures (BEAS-2B and HBEC) with every respiratory virus used. But interestingly no up regulation of these interferon types was seen. So probably these alpha-interferon types are constitutively expressed in epithelial cell cultures but not induced by respiratory viruses which were used. And according to the ELISA data none of these alpha-interferon types are produced by epithelial cell lines.

The level of detection of alpha-interferons with second primer (types 2,4,5,8,10,14,17,21) pair is quite different. It is induced in epithelial cell lines most often at 8 hours after the infection. This primer pair detects interferon-alpha 4 which can be the reason for this induction. But the story with protein is the same - it is not produced in epithelial cells.

It is also interesting that in HBEC the level of induction of alpha-interferons detected by second primer pair is lower when the cells are infected with influenza virus than they are infected by rhinoviruses 16 and 1B. Which indicates that influenza virus downregulates the induction of alpha interferons in epithelial cells.

So alpha-interferons are expressed, induced but not produced in epithelial cells after respiratory virus infection.

In the study interferon-beta was induced later than IFNa.2 alpha-interferons by respiratory viruses used.

Interestingly interferon-beta is differently produced in various epithelial cell lines. In BEAS-2B cells it is produced by 24 hours over rhinovirus infection thus in HBEC it is not produced after infection with neither rhinovirus 16 nor with rhinovirus 1B and influenza virus.

IL-29 mRNA is induced in both epithelial cell lines by 8 and 24 hours.

In epithelial cells mRNA of IFNα.1 types of alpha-interferons is expressed and not induced and neither produced. mRNA of IFNα2 (which contain interferon alpha-4) types of interferon-alpha is induced by various rhinoviruses in studied epithelial cell line but no interferon-alpha is produced by epithelial cell lines.

So in epithelial cells differences in kinetics of type 1 and type III interferons can be seen. In BEAS-2B cells over the rhinovirus 16 infection alpha-interferons are expressed by 8 hours and then go down. Whilst interferon-beta peaks only at 24 hours. IL-29 mRNA in BEAS-2B cells starts rising at 8-hour time point and goes even higher at 24 hour time-point.

In HBEC infected with rhinovirus 16 alpha-interferons are up regulated by 8 hours and stay the same by 24 hour time point. Interferon-beta is up regulated by 24 hours as well as IL-29.

HBEC infected with rhinovirus 1B has the same kinetics of type 1 and type III interferon expression. They are up regulated at the same time points, which demonstrates that the kinetics of rhinovirus up regulation doesn't depend on rhinovirus type and probably indicates that they have the same induction pathway.

Although the level of induction of type 1 and type III interferons by influenza virus is lower in comparison to rhinovirus infection it has nearly the same kinetics: alpha-interferons also go up by 8 and 24 hours, beta-interferon and IL-29 peak at 24 hours. This also not variable from data obtained with rhinovirus infection.

### UV- data

It has been demonstrated that alpha-interferons are expressed in epithelial cells, Moreover some of them are induced by various respiratory viruses. But in both studied respiratory epithelial cell cultures they are not produced. Alpha-interferons are vital antiviral factors, as they induce hundreds of interferon inducible genes with antiviral properties. Some epithelial cell lines are able to produce alpha-interferons under certain conditions and stimuli, but it seems that either respiratory epithelium is not able to produce alpha-interferons, or respiratory viruses are not potent inducers of alpha-interferon production in respiratory epithelium. And it seems that the most important producers of alpha-interferons during respiratory virus infections are plasmacytoid dendritic cells (Cella, M., D. Jarrossay, F. Facchetti, O. Alebardi, H. Nakajima, A. Lanzavecchia, and M. Colonna. 1999. Plasmacytoid monocytes migrate to inflamed lymph nodes and produce large amounts of type I interferon. Nat. Med. 5:919-923.) and macrophages.

Interestingly beta-interferons are induced and produced in some epithelial cell lines, such as BEAS-2B.

The same is observed with IL-29, but induction of this type III interferon is earlier, to a greater level and more sustained than induction of the type I interferons.

### Example 4: Further experimental procedures

We provide below further experimental procedures used to derive the data presented herein.

### Outline Of Experimental Design And Techniques

RV16 experimental infections were induced in RV16 seronegative asthmatic and normal subjects. Baseline, acute infection and convalescent (6 week) blood, nasal, sputum and bronchoalveolar sampling were carried out to investigate baseline status, the acute phase of illness, and the degree of persistence. 17 normal, non-atopic and 11 atopic, mild asthmatic adults were recruited. Clinical and atopic status were defined by questionnaire, skin prick testing, serum IgE and lung function testing including histamine PC20. The asthmatic group were required to have a histamine PC₂₀ <8mg/ml, the normal group >8mg/ml. Individuals taking inhaled/oral steroids were excluded. Subjects were free of common cold symptoms for 6 weeks before commencing the study. Samples were taken according to established protocols developed in previous studies. These included blood, nasal lavage (NL), and bronchoalveolar lavage (BAL). Baseline samples were taken 2wks prior to infection. Following inoculation on day 0, volunteers attended on days 3, 4 and 7 (at the height of cold symptoms) for further samples and lung function tests. Volunteers also attended daily from day 0 (prior to inoculation) to day 8 and on day 11 for NL to determine viral load. A third set of samples and lung function tests were performed at 6 weeks.

### Experimental infection with RV16

Protocols for experimental virus infection have been described in previous studies (Bardin et al., (1996) European Respiratory Journal 9, 2250-2255; Fraenkel et al., (1995) Am. J. Respir. & Crit. Care Med. 15, 879-886; Bardin et al., (1994) Clin Exp. Allergy 24, 457-464).,Details regarding preparation and safety testing of the RV16 inoculum have been published (Bardin et al (1996) *supra).* Experimental infection was induced using 10000 TCID₅₀ RV 16 on day 0 by nasal spray, with a DeVillbiss 286 atomizer. 2 aliquots of 500 µl (2500 TCID₅₀) were applied to each nostril. Inoculation was carried out in a specified clinical room at the end of the clinic day. Subjects avoided individuals with respiratory infection to minimise risk of a non-RV16 infection during the study. Infection was confirmed by culture of NL in HeLa cells for RV, or by positive serology. RNA was extracted from NL and BAL cell pellets and analysed by PCR for RV. Taqman PCR was used to quantify viral RNA. Co-infection with additional respiratory viruses including alternative RV serotypes was excluded by PCR for other viruses and by neutralisation of cultured rhinoviruses with RV 16 specific antisera.

### Criteria for virological confirmation of RV16 infection

Successful experimental RV16 infection was confirmed by at least one of the following virological tests: Positive standard or Taqman RT-PCR for RV from upper (nasal lavage) and/or lower (induced sputum, bronchoalveolar lavage) airway samples; a rise in serum neutralising antibodies to RV16 6 weeks after inoculation of at least 4-fold (in the case of subjects in this study who were seronegative at baseline a titre of at least 1:4 was considered satisfactory); positive culture ofRV from nasal lavage in HeLa cells with, after repeat passaging of virus to obtain a satisfactory concentration as determined by titration assay, clear RV cytopathic effect on HeLa cell monolayers with neutralisation by guinea pig specific RV16 antiserum. Standard picornavirus RT-PCR was performed on nasal lavage collected on the day with the peak Taqman RT-PCR viral load. Restriction enzyme analysis was then carried out to confirm the identity of positive picornavirus as RV and not enterovirus. Similarly, viral culture was performed using nasal lavage from the peak day on the basis of the Taqman results.

### Collection of clinical data

Subjects recorded cold and chest symptom scores daily during an initial screening phase and from baseline, starting 2 weeks prior to the baseline bronchoscopy through to convalescence, finishing 2 weeks after the convalescent bronchoscopy performed 6 weeks after the experimental RV16 infection. In addition to symptom scores the subjects noted the timing and amount of medication such as inhaled bronchodilators required. Lung function was assessed by 2 methods. Firstly, subjects performed home spirometry using a portable handheld spirometer twice daily, in the morning immediately after waking and last thing at night. Secondly, Histamine PC20 tests were used to assess bronchial hyperreactivity on screening, at baseline, at day 6 post inoculation and in convalescence.

*Diary cards for symptom scores* / *medication usage* / *spirometry recording* Symptom assessment was by questionnaire for 2 weeks prior to, during and for 6 weeks after infection. The cold score was based on that of earlier common cold studies (Jackson et al (1958) Arch Int Med 101:267-278). Symptoms (sneezing, headache, malaise, chilliness, nasal discharge, nasal obstruction, sore throat, cough, fever) were graded 0-3. A clinical cold was defined by a minimum cumulative score of 14 over 6 days (>20=severe cold) plus a subjective impression of cold or rhinorrhoea. Chest score symptoms included: cough on waking; wheeze on waking; daytime cough; daytime wheeze; daytime shortness of breath; nocturnal cough, wheeze or shortness of breath.

### Analysis of clinical symptom scores

To facilitate analysis of the clinical data the experimental infection protocol was divided up into separate stages. In addition to calculation of daily scores for individual symptom and total cold or chest scores, 2 week scores were calculated to allow for statistical analysis of the effects of RV infection on symptoms. It was decided to examine 2 week stages because following RV inoculation excess symptoms lasted for up to 2 weeks. The pre-baseline or screening stage was the 2 weeks up to the beginning of the baseline stage, the pre-convalescent stage was the 2 weeks immediately before the convalescent bronchoscopy. Neither of these 2 stages contained bronchoscopy. The baseline, acute infection and the convalescent stages all contain bronchoscopy on the 4^{th} day of that 2 week block. To examine the effects of the RV16 infection on symptoms daily and 2 week excess symptom scores were calculated by subtracting the scores obtained during the baseline stage from the corresponding days of the acute infection stage to correct for the effects of undergoing bronchoscopy, which itself may result in cold and chest symptoms and in short lived changes in lung function.

### Lung Function Testing

Lung function testing was performed according to BTS/ARTP guidelines (Anonymous. (1994). Guidelines for the measurement of respiratory function. Recommendations of the British Thoracic Society and the Association of Respiratory Technicians and Physiologists. Respiratory Medicine 88:165-194). Subjects used a portable spirometer at home, the microDL (MicroMedical) morning and evening. Data was analysed using Spida software. In the lung function laboratory, and for bronchodilator reversibility, sputum induction and histamine challenge subjects used a Vitalograph Dry Wedge Bellows Spirometer. To facilitate comparison of changes in the 2 groups during the experimental infections firstly the % change in FEV1 from the mean obtained during the screening stage was calculated for each subject on the days following RV 16 inoculation and secondly this was corrected for changes seen following bronchoscopy in the corresponding baseline days.

### Histamine Challenge

Histamine challenge was performed according to ERS guidelines (Sterk et al (1993) Airway responsiveness. Standardized challenge testing with pharmacological, physical and sensitizing stimuli in adults. Report Working Party Standardization of Lung Function Tests, European Community for Steel and Coal. Official Statement of the European Respiratory Society. [Review] European Respiratory Journal - Supplement 16:53-83) using the 2 minute tidal breathing method. Bronchial hyperreactivity was assessed at baseline, day 6 post-infection and at 6 weeks.

### Skin prick testing

Atopy was determined by skin prick testing to common aeroallergens: grass pollen; house dust mite; cat dander; dog hair; aspergillus fumigatus; cladosporium herbarum; alternaria altemata; silver birch; 3 trees; nettle pollen. Positive histamine / negative diluent controls were included. 1 positive reaction (wheal 3 mm greater than negative control) was considered diagnostic of atopy.

### Nasal lavage

NL was performed for: standard and Taqman RT-PCR for RV viral load; to confirm infection by effects on HeLa cell culture. 2.5ml sterile normal saline was instilled into each nostril using a soft plastic pippete. Lavage was collected into a sterile petri dish, homogenised then aliquotted for storage at -80C.

### Peripheral blood analyses

50ml blood was collected in heparinised tubes, diluted 1:1 with PBS then layered over lymphoprep. After centrifugation 2500rpm 30mins mononuclear cells were transferred to a single polypropylene tube and washed in RPMI-1640 10%FCS. The cell suspension was diluted 1:10 in 0.1 % trypan blue for counting and assessment of viability by haemocytometer. Cells were resuspended in appropriate culture medium at the required cell density for subsequent experiments.

### Serum separation

10ml blood was collected in a plain vacutainer tube, placed at 37C 4h to clot before centrifuging 2000rpm 15mins. Serum was aliquotted for storage at -80C for subsequent analysis for the presence of RV16 neutralising antibody.

### Bronchoscopy

Bronchoscopies were performed according to BTS guidelines (British Thoracic society Bronchoscopy Guidelines Committee. 2001. British Thoracic Society guidelines on diagnostic flexible bromnchoscopy. Thorax 56:i1-i21) in the endoscopy unit at St Marys Hospital. Subjects were monitored by a separate physician or nurse. FEV1 was recorded prior to and after the bronchoscopy. A Keymed P100 bronchoscope was used with fenestrated forceps (Keymed FB-19C-1) and 3mm sheathed brushes (Keymed BC-16C). BAL was performed by instillation of sterile normal saline (room temperature) into the right middle lobe bronchus in 8x30ml aliquots with a 10s dwell time, aiming for 80% recovery. At baseline and 6 weeks BAL was obtained from the medial segment right middle lobe, at day 4 from the lateral segment to minimise effects of the previous BAL. BAL was collected in a single plastic chamber and transferred immediately to polypropylene tubes on ice for transport to the laboratory.

### RV16 serology

RV16 serology was performed at screening, baseline, d0 and 6wks post infection by microneutralisation test for neutralising antibody to RV16 utilising HeLa cell monolayers in 96 well plates. Doubling dilutions of sera (50µl) were made from 1:2 to 1:128. 50µl diluted stock virus containing 100TCID₅₀ was added and the plate shaken at room temperature for 1h. 100µl freshly stripped HeLa cells 2x10⁵ cells/ml were added and plates incubated at 37C. Serum (cells + serum at 1:2 dilution), cell (cells, no serum, no virus) and virus (cells, no serum, stock virus) controls were included. Cytopathic effect (CPE) was read after 2-3 days. Antibody titre was defined by the greatest serum dilution completely neutralising viral CPE. Seroconversion was defined in seronegative subjects as a convalescent titre of RV 16 neutralising antibodies of 1:4 or greater.

### Virus culture from clinical samples

The presence of RV in nasal lavage was determined by culture. This was initially performed at 37C and if negative repeated at 33C. Virus was cultured by adding sample to a small volume of medium containing antibiotics and covering semiconfluent HeLa cells in a T25 flask, shaking at room temperature for 1h, then adding additional medium and observing for CPE. If absent cells were lysed by 2 freeze/thaw cycles at 5 days and the clarified supernatant was added to fresh HeLa cells. If after 5 passages no CPE was observed virus was considered absent. Confirmation of cultured virus as RV16 involved a microneutralisation assay with RV16-specific sera (ATCC - titre 1:600). RV titre in culture supernatant was estimated by titration assay. Then in a 96-well plate 50µl of diluted supernatant containing 100TCID₅₀ of virus was added to an equal volume of medium containing 2-fold serial dilutions of the specific RV16 antisera from 1:20 to 1:1280. The assay included positive (stock RV 16) and cell (no virus) controls.

### RNA extraction from stored clinical samples and reverse transcription using random hexamer primers

RNA was extracted from samples using the QIAamp viral RNA mini kit (Qiagen) and reverse transciption performed using the omniscript RT kit (Qiagen) and random hexamer primers as per the manufacturer's instructions.

### Standard PCR for picornaviruses

RV RT-PCR was performed from cDNA produced by RT using random hexamer primers. PCR was performed using the Perkin Elmer 9600 GeneAmp PCR system using the published method (Johnston et al (1993) Journal of Clinical Microbiology 31:111-117) utilising the OL26/OL27 primer pair. The 380bp picornavirus specific amplicon generated was visualised by ethidium bromine staining after electrophoresis on a 2% agarose gel and photographed by polaroid camera. RV amplicons were distinguished from those of enteroviruses by restriction digestion using Bgl I (Papadopoulos et al (1999) Journal of Virological Methods 80:179-185).

### PCR for additional respiratory viruses

The presence of respiratory viruses other than RV was excluded by PCR for *Mycoplasma* and *Chlamydia pneumoniae,* adenoviruses, respiratory syncytial virus, influenza AH 1 / AH3 / B, parainfluenza 1-3, coronaviruses 229E and OC43. cDNA for these PCRs was produced by random hexamer RT. The protocols for these additional PCRs are previously published (Seemungal et al. (2001) American Journal of Respiratory & Critical Care Medicine 164:1618-1623)

### Taqman RT-PCR for picornavirus

Taqman RT-PCR was used to detect picornavirus in NL and BAL stored unprocessed after sampling at -80C. RNA was extracted from samples using the QIAamp viral RNA mini kit (Qiagen) and reverse transciption performed using the omniscript RT kit (Qiagen) and random hexamer primers as per the manufacturer's instructions. PCR was performed using the PE Biosystems ABI Prism 7700 sequence detection system with AmplitaqGold DNA polymerase, a picornavirus specific primer pair (forward oligo 5'-GTG AAG AGC CSC RTG TGC T-3', reverse oligo 5'-GCT SCA GGG TTA AGG TTA GCC-3') and a FAM/TAMRA labelled picornavirus probe (FAM-TGA GTC CTC CGG CCC CTG AAT G-TAMRA).

A master mix was made up consisting of Qiagen quantitect probe mix, forward primer (50nM) reverse primer (300nM), probe (100nM) and Rnase inhibitor. 23µl of PCR master mix was added to 2µl cDNA in each tube of the 96 well Taqman plate. Thermal cycling and detection of fluorecent PCR product was carried out using the PE Biosystems ABI Prism 7700 sequence detection system. The thermal cycle conditions used were: 50C 2min; 95C 10min; then 45 cycles x 95C 15s / 55C 20s / 72C 40s. The Taqman RT-PCR methodology had been optimised by collaborators at Viropharma (Pevear et al (1999) Antimicrobial Agents & Chemotherapy 43:2109-2115). Fluorescence data was collected for each cycle and the cycle number (Ct) at which fluorescence rose above threshold was determined. Negative extraction (water), negative PCR (template only) and positive extraction (RV16 stock) were included. A standard curve was produced by including in the Taqman plate tubes containing 2µl of RV plasmid serially diluted 10 fold from 10⁸ to 10⁰ copies / 2µl. After PCR each plasmid generates 1 copy dsDNA. Results were expressed for each sample in terms of copies/ml for NL and BAL by reference to the standard curve and taking into account both dilution factors inherent in processing to RNA and cDNA and the "double fluorescence" produced by each copy of dsDNA plasmid.

### Statistical analysis

Symptom scores, lung function, PC₂₀ values, virus load, cytokine and chemokine concentration and leukocyte numbers were compared within subjects to determine differences induced between baseline and the acute cold, and persistence of changes into convalescence. Intra-subject differences were analysed using Wilcoxon's test. Differences between normal and asthmatic groups were analysed using Mann Whitney's test at each phase of the study. Correlations between clinical illness severity, virus load, leukocyte counts and cytokine/chemokine concentrations were examined using Spearman's rank correlation to investigate possible causal relationships for these factors regulating the altered response in asthma.

### BAL ex vivo cultures

BAL cells from the bronchoscopy performed at baseline prior to experimental infection have been cultured for 48h ex vivo in polypropylene tubes prior to harvesting of supernatant for cytokine production and cells for RNA, culture conditions including the following: medium only, medium +RV16 5MOI, medium +RV16 filter control, medium +LPS 0.1 µg/ml, medium +PHA 1µg/ml, medium +allergen 5000 ISQ. On harvesting cells were vortexed briefly before centrifugation 1500rpm 10mins. Supematent was aliquotted and stored at -80C for subsequent analysis by ELISA. 1ml of trizol was added to lyse the cells before storage at -80C for subsequent analysis by RT-PCR.

### Example 5: Correlation of IFNλ protein levels with clinical indicators of infection

The correlation between IFNλ protein levels and clinical indicators of respiratory infection was further investigated. The data is presented in Figure 22. The methods used are outlined above in Example 4.

Figure 22 shows IFNλ levels in bronchoalveolar cell supernatants, stimulated ex *vivo* with rhinovirus.

Figure 22 (a) shows the quantity of IFNλ protein in the supernatant of *ex vivo* RV-stimulated bronchoalveolar cells from normal and asthmatic subjects. It is clear from this data that cells isolated from asthmatic subjects produce much lower amounts of IFNλ protein than cells isolated from non-asthmatics subjects. Hence, bronchoalveolar cells from asthmatic subjects do not produce as much IFNλ protein when infected with RV than bronchoalveolar cells from normal subjects.

Figure 22 (b) illustrates the relationship between IFNλ protein levels in patients infected with RV and the "cold score" of the patients when infected in vivo with rhinovius 2 weeeks later. "Cold score" is a clinical measure of the severity of the respiratory viral infection, as discussed above in Example 5. It is clear that patients that have lower IFNλ protein levels have a higher "cold score" than patients with more IFNλ protein. Accordingly, the data demonstrates the correlation between IFNλ protein levels and the severity of clinical indicators of respiratory viral infection. Hence IFNλ protein may be of use in the treatment of respiratory disorders.

Figure 22 (c) shows the relationship between lung capacity (as measured using FEV 1 values) in patients infected with RV and IFNλ protein levels. It is clear that there is a correlation between the reduction in FEV1 and the level of IFNX protein in the patients. Thus patients with lower IFNλ production at baseline suffer more severe airway obstruction when infected with rhinovirus 2 weeks later, hence IFNλ may be used in reducing severity of asthma excerbations.

Figure 22 (d) shows the amount of RV16 RNA in BAL cells (therefore lower airway virus load) taken during an *in vivo* infection with rhinovirus correlated with IFNλ protein production in BAL cells taken at baseline 2 weeks prior to the *in vivo* infection at baseline. Again, there is a correlation between the amount of RV16 RNA levels and IFNλ protein levels: the more IFNλ protein, the less RV16 RNA is present. Thus IFNλ may be used to diminish virus load in the lower airway thereby preventing/ameliorating virus induced asthma exacerbations.

The data presented in Figure 22 provides correlations between lambda production and virus load, lung function and other clinical indicators of outcome. This data clearly shows the important biological role for IFNλs in protecting against viral induced exacerbations.

## Claims

1. An agent selected from: (a) one or more interferon lambda (IFN-λ) polypeptides or (b) a polynucleotide or polynucleotides which express one or more IFN-λ polypeptides in target bronchial epithelial cells, for use in the treatment of viral-induced exacerbation of a respiratory disorder selected from asthma and chronic obstructive pulmonary disease (COPD).

2. An agent for use as claimed in claim 1 wherein said respiratory disorder is asthma.

3. An agent for use as claimed in claim 1 or claim 2 wherein said viral-induced exacerbation is caused by infection with a virus selected from the group consisting of rhinovirus, RSV and influenza virus.

4. An agent for use as claimed in any one of claims 1 to 3 which is one or more IFNλ polypeptides selected from the group consisting of IFNλ1, IFNλ2 and IFNλ3.

5. An agent for use as claimed in any one of claim 1 to 3 which is one or more polynucleotides which express in target bronchial epithelial cells one or more IFNλ polypeptides selected from the group consisting of IFNλ1, IFNλ2 and IFNλ3.

6. An agent for use according to any one of the preceding claims formulated for administration by airway delivery.

7. An agent for use according to any one of claims 1 to 6, As a combined product with an inhaled corticosteroid, wherein said agent and said corticosteroid are to be administered simultaneously, separately or sequentially to treat viral exacerbation of a respiratory disorder selected from asthma and COPD.

## Patentansprüche

1. Wirkstoff, der ausgewählt wird, aus:
(a) einem oder mehreren Interferon Lambda (IFN-λ) Polypeptiden oder
(b) einem Polynucleotid oder Polynucleotiden, die ein oder mehrere IFN-λ Polypeptide in bronchialen Ziel-Epithelialzellen ausdrücken;
zum Einsatz in der Behandlung einer viral induzierten Verschlimmerung einer Atemwegserkrankung, die aus Asthma und einer chronisch obstruktiven Lungenerkrankung (COPD)ausgewählt wird.

2. Wirkstoff zum Einsatz nach Anspruch 1, wobei es sich bei der Atemwegserkrankung um Asthma handelt.

3. Wirkstoff zum Einsatz nach Anspruch 1 oder Anspruch 2, wobei die genannte viral induzierte Verschlimmerung durch eine Infizierung mit einem Virus bewirkt wird, das aus der Gruppe ausgewählt wird, die das Rhinovirus, RSV und das Influenzavirus umfasst.

4. Wirkstoff zum Einsatz nach einem der Ansprüche 1 bis 3, wobei es sich um ein oder mehrere IFNλ Polypeptide handelt, die aus der Gruppe ausgewählt werden, die IFNλ1, IFNλ2 und IFNλ3 umfasst.

5. Wirkstoff zum Einsatz nach einem der Ansprüche 1 bis 3, wobei es sich um ein oder mehrere Polynucleotide handelt, die in bronchialen Ziel-Epithelialzellen ein oder mehrere IFNλ Polypeptide ausdrücken, die aus der Gruppe ausgewählt werden, die IFNλ1, IFNλ2 und IFNλ3 umfasst.

6. Wirkstoff zum Einsatz nach einem der vorstehenden Ansprüche, mit einer Formulierung für eine Verabreichungmittels Luftwegzufuhr.

7. Wirkstoff zum Einsatz nach einem der Ansprüche 1 bis 6 als ein kombiniertes Produkt mit einem inhalierten Corticosteroid, wobei der genannte Wirkstoff und das genannte Corticosteroidgleichzeitig, separat oder sequentiell verabreicht werden, um eine virale Verschlimmerung einer aus Asthma und COPD ausgewählten Atemwegserkrankung zu behandeln.

## Revendications

1. Agent choisi parmi :
(a) un ou plusieurs polypeptidesd'interféron lambda (IFN-λ) ou
(b) un polynucléotide ou des polynucléotides qui expriment un ou plusieurs polypeptides IFN-λ dans des cellules épithéliales bronchiques cibles,
pour une utilisation dans le traitement de l'exacerbation d'origine virale d'un trouble respiratoire choisi parmi l'asthme et la broncho-pneumopathie chronique obstructive (BPCO).

2. Agent pour une utilisation selon la revendication 1, dans lequel ledit trouble respiratoire est l'asthme.

3. Agent pour une utilisation selon la revendication 1 ou 2, dans lequel ladite exacerbation d'origine virale est causéepar une infection par un virus choisi dans le groupe constitué du rhinovirus, du virus respiratoire syncytial et du virus de la grippe.

4. Agent pour une utilisation selon l'une quelconque des revendications 1 à 3, qui est un ou plusieurs polypeptides IFNλ choisis dans le groupe constitué de IFNλ1, IFNλ2 et IFNλ3.

5. Agent pour une utilisation selon l'une quelconque des revendications 1 à 3, qui est un ou plusieurs polynucléotides qui expriment dans des cellules épithéliales bronchiques cibles un ou plusieurs polypeptidesIFNλ, choisis dans le groupe constitué de IFNλ1, IFNλ2 et IFNλ3.

6. Agent pour une utilisation selon l'une quelconque des revendicationsprécédentes, formulé pour une administration par des voies respiratoires.

7. Agent pour une utilisation selon l'une quelconque des revendications 1 à 6, comme un produit combiné avec un corticostéroïde en inhalation, dans lequel ledit agent et ledit corticostéroïde doivent être administréssimultanément, séparément ou séquentiellement pour traiter l'exacerbation virale d'un trouble respiratoire choisi parmi l'asthme et la BPCO.
